# EUROPEAN PATENT APPLICATION

(11) **EP 4 248 898 A1**
(43) Date of publication of application: **27.09.2023**
(21) Application number: 23163979.0
(22) Date of filing: 24.03.2023
(51) Int. Cl.: A61B 18/20

(54) **SYSTEMS FOR CONTROLLING THERAPEUTIC LASER TREATMENT BASED ON A COOLING TO HEATING RATIO**

(30) Priority: 25.03.2022 US 202263324004 P; 23.10.2022 US 202217971637
(71) Applicant: Cutera Inc., Brisbane, CA 94005 (US)
(72) Inventor: KARAVITIS, Michael, San Pedro (US); KOTHARE, Amogh, Fremont (US); MOELLER, Soenke, Berkeley (US)
(74) Representative: Grünecker Patent- und Rechtsanwälte PartG mbB

(57) **Abstract**

Dermatological systems and methods for providing a therapeutic laser treatment wherein the magnitude of heat power or energy applied to a target skin area via a therapeutic laser pulse is based on the magnitude of cooling power or energy applied to the target skin area during a cooling of the skin prior to treatment with one or more therapeutic laser pulses.

## Description

### Cross-Reference to Related Applications

**This** invention claims the benefit of priority to U.S. Provisional Application Serial No. 63/324,004 filed March 25, 2022, entitled "Systems and Methods for Controlling Therapeutic Laser Use Based on a Heating to Cooling Ratio".

### Background of the Invention

This invention relates to electromagnetic radiation-based medical treatment systems, and more specifically to systems and methods for controlling heating and cooling of a target skin area in the treatment of dermatological conditions. The invention includes controlling the ratio of heating and cooling power for such treatments.

**A** variety of dermatological conditions are treatable using electromagnetic radiation (EMR). Sources of EMR in dermatological treatment systems may include, without limitation, lasers, flashlamps, and RF sources. Lasers are frequently used as an EMR source to treat a range of conditions including acne vulgaris, abnormal pigmentation, vascular skin conditions (*e.g*., spider veins), wrinkles and fine lines, dyschromia, and many others. Both pulsed and continuous-wave (CW) laser systems have been used, although pulsed lasers are more commonly employed.

Many dermatological EMR systems use a laser to photo-thermally damage a target tissue while preserving surrounding or adjacent non-targeted tissues or structures. The principle of selective photothermolysis, which involves thermally damaging a target tissue to promote a healing response, has led to a variety of laser applications as standard of care in many medical fields such as ophthalmology and dermatology.

Thermal damage during photothermolysis involves raising the temperature of the target tissue to a damage threshold temperature for a specified time period. For a desired level of thermal damage, there is a tradeoff between the temperature of the target tissue and the time it must remain at that temperature. Similar thermal damage may be achieved using a lower temperature if the time at the temperature is increased; if a higher temperature is used, a shorter time at the higher temperature can achieve an equivalent level of thermal damage. To avoid thermal damage to non-targeted tissue, it is desirable to limit the heating time to the thermal relaxation time (TRT) of the target tissue. TRT is the time required for the target to dissipate about 63% of the thermal energy received from the pulse. It is related to the size of the target chromophore, and may range from a few nanoseconds for small chromophores such as tattoo ink particles, to hundreds of milliseconds for large chromophores such as leg venules. Accordingly, in many cases, a damage threshold temperature for a desired level of thermal damage may be selected based on the TRT of the target tissue. For example, depending upon parameters such as the laser power, fluence, spot size, etc. in a given system, a damage threshold temperature for a desired level of photothermolysis at time periods approximately equal to the TRT may be selected. The laser power may be adjusted, e.g., by a user or automatically, based upon one or more characteristics of a patient's skin (*e.g*., color or tone, thickness of various skin layers, etc.).

Photothermolysis can be achieved when three conditions are met: 1) the wavelength of the laser is chosen to have a preferential absorption in the target chromophore(s) over non-target chromophores in the target tissue; 2) the pulse duration of the laser should be equal to or less than (=<) the thermal relaxation time (TRT) of the target chromophore(s) in the target tissue; and 3) the laser fluence (*i.e*., energy per unit area) must be sufficient to exceed the thermal damage threshold of the target chromophore(s) in the target tissue. Together, these principles permit laser systems to be developed that deliver energy at specific wavelengths, pulse durations, and fluences to provide controlled energy to damage target tissue while leaving non-targeted surrounding tissues and structures substantially unaffected. If the laser pulse duration is less than the TRT of the target tissue, no significant heat can escape into non-target structures, and damage to non-target structures is limited.

Selectivity as well as overall safety would be improved if the temperature of the skin could be dynamically controlled. In particular, most laser-based dermatological treatment systems do not provide reliable control of the skin temperature during treatment, since pulse durations and the number of pulses applied to a target treatment area are typically selected by a system user and maintained for a given treatment session until manually changed by the user (*e.g*., a laser technician, physician, *etc.).* There is a need for laser treatment systems providing better skin temperature control. Some embodiments of this disclosure achieve this by using the actual skin temperature to provide feedback to dynamically control the temperature during treatment.

Ideally, thermal damage is highly localized to only the particular target tissue (*e.g*., a particular skin layer, or particular structures such as chromophores within a skin layer at a particular location), with nearby non-targeted tissues/structures remaining unaffected and available to facilitate the healing response in the target tissue. However, the structural complexity of the skin, which includes a variety of layers each having unique structural and functional characteristics, has limited the development of effective EMR-based treatments for many skin conditions.

Achieving a controlled thermal damage level to, target structures within skin tissue by laser radiation is complicated by a variety of intrinsic and extrinsic factors. Intrinsic factors include, without limitation, the depth of the target structure(s) within the target tissue and the associated absorption of light by non-targeted structures overlying the target (which may involve a plurality of intervening structures each having different light absorption and thermal characteristics), the scattering of light within the skin above the target, the TRT of the target structure and intervening non-target structures, and the removal (or non-removal) of heat by blood flowing through dermal and subdermal vasculature, including a superficial and deep vascular plexus as well as glomus bodies. Extrinsic factors include, without limitation, wavelength, pulse width, power, fluence, spot size, and other characteristics of the laser used to treat the target tissue or structure(s).

Acne vulgaris, more commonly referred to simply as acne, is the most common reason for office visits to US dermatologists. Over 60 million Americans suffer from acne. Treatment options include topical applications such as disinfectants , retinoids (*e.g*., Retin-A), antibiotics (*e.g*., clindamycin and erythromycin), as well as ingested compounds such as antibiotics, hormonal treatments (*e.g*., birth control pills), isotretinoin (Accutane), and optical treatments such as laser treatments, which have the benefit of avoiding the side effects and inconvenience of pharmaceuticals and topical treatments but may have limited effectiveness for a variety of reasons including the previously noted problems of skin tissue complexity and the limitations of existing laser systems. More recently, nanosphere particles have been deposited into skin pores and/or follicles, then heated with laser light to treat acne. Photodynamic therapies, in which an agent is applied to the skin to increase its sensitivity to light, have also been used with laser or other light (e.g., blue light) to treat acne.

There is a need for improved laser systems and methods having greater efficacy for treating acne and other medical conditions, which the present disclosure provides. In one aspect, the present disclosure provides systems having a defined ratio of cooling power to heating (i.e., laser) power to limit damage to tissues overlying a target structure (e.g., a sebaceous gland in the case of acne). In one aspect, the disclosure provides systems and methods providing contact cooling having a desired cooling power (i.e., rate of energy removal) from a skin area including target skin area(s) receiving one or more laser pulses and adjacent areas. The desired cooling power is provided to achieve a desired ratio of cooling to the heating power of the laser being applied to the skin. Having a defined ratio of cooling to heating power becomes highly important when the target structure is deeper in the skin. By providing a high ratio of cooling power compared to prior art systems, the present disclosure provides for systems in which skin areas immediately adjacent to the target skin area receiving the laser energy can be used as a cooling heat sink to remove heat from non-target structures overlying the deeper target structure, such as the epidermis and/or dermal layers overlying a sebaceous gland.

For deeper target structures such as sebaceous glands, which often range from 0.3 - 2.0 mm (more commonly 0.5-1.0 mm) below the outer surface of the epidermis, damage to overlying tissue structures is difficult to control or limit, since the laser energy must pass through those tissue structures before reaching the target tissue structures. The overlying tissue structures absorb energy depending upon their respective depths and absorption coefficients, and may receive undesired damage as the laser light penetrates to the target tissue structures. In some instances, the target structures are either sufficiently shallow, or the treatment temperature to which the target structures are raised is sufficiently low, that the heating of overlying structures may not cause excessive damage. Even where the risk of overheating the overlying structures of a relatively deep target is minimal, accurate temperature control of the target structure may be poor, resulting in overheating or underheating of the target structure, patient discomfort, or both.

The skin surface may be cooled to limit the temperature increase (and consequent damage) to non-targeted overlying structures, as well as to limit discomfort and pain. However, existing systems lack precise control of the cooling process, and do not provide sufficient cooling power to achieve both a desired level of photothermal damage to deeper target structures and minimize damage to non-targeted overlying structures. In many cases, the skin surface is cooled either too much-in which case the deeper target structure fails to reach a temperature damage threshold-or too little, in which case non-target overlying structures are damaged and the deeper target structure may be excessively damaged. There is a need for laser-based treatment systems having improved temperature control of the cooling process to ensure that target structures reach a desired thermal damage temperature and that thermal damage to non-targeted structures is minimized or controlled to an acceptable level.

There is a need for dermatological laser systems that are able to efficiently treat a variety of medical conditions to achieve these goals.

### Summary

In one embodiment, the invention comprises a method of treating the skin of a patient with a therapeutic laser pulse, the method comprising: applying a contact cooling element comprising a cooling window to a first skin area of the patient; b) cooling at least a target skin area within the first skin area prior to initiating an application of a therapeutic laser pulse to the target skin area, wherein the cooling power applied to the target skin area is a first preselected setpoint; and c) applying a therapeutic laser pulse to the target skin area through the cooling window at a first timepoint wherein heating power applied to the target skin area by the therapeutic laser pulse is a second preselected setpoint, the first preselected setpoint having a ratio in the range of about 15-25% of the second preselected setpoint.

In another embodiment, the invention comprises a method of treating the skin of a patient with a therapeutic laser pulse, the method comprising: applying a contact cooling window to a first skin area of the patient, wherein the contact cooling element is coupled to a handpiece adapted to be held by a user; cooling at least one target skin area within the first skin area using the contact cooling window, wherein the cooling energy is provided using a contact cooling unit thermally coupled to the contact cooling window and having a cooling power of at least 4 Watts (W); applying one or more therapeutic laser pulses to the at least one target skin area through the cooling window, wherein the one or more therapeutic laser pulses are generated using a laser source having a laser source power of at least 20 Watts, wherein the ratio of the cooling power and the laser source power is at least 20%.

In another embodiment, the invention comprises a method of treating the skin of a patient with a therapeutic laser pulse, the method comprising: applying a contact cooling window to a first skin area of the patient, wherein the contact cooling element is coupled to a handpiece adapted to be held by a user; cooling at least one target skin area within the first skin area using the contact cooling window, wherein the cooling energy is provided using a contact cooling unit thermally coupled to the contact cooling window and having a cooling power of at least 4 Watts; applying one or more therapeutic laser pulses to the at least one target skin area through the cooling window, wherein the one or more therapeutic laser pulses are generated using a laser source having a laser source power of at least 20 Watts, wherein the ratio of the cooling power and the laser source power is at least 15%.

In another embodiment, the invention comprises a system of treating the skin of a patient with a therapeutic laser pulse, the system comprising: a laser source for providing a plurality of therapeutic laser pulses for application to one or more target skin areas within a first skin area of the patient; a handpiece comprising a handpiece body comprising an aperture, the handpiece body optically coupled to the laser source and having a pulse delivery region adapted to deliver the therapeutic laser pulses to the one or more locations within the first skin area through the aperture; a cooling element adapted to provide cooling to the first skin area based on a cooling power; and a controller for controlling an application of one or more therapeutic laser pulses to at least a portion of the first skin area, wherein the one or more therapeutic laser pulses are generated using the laser source, wherein the laser source has a laser source power of at least 20 Watts, and wherein the ratio of the cooling power and the laser source power is at least 20%.

### Brief Description of the Drawings

Figure 1 is a cross-sectional illustration of skin tissue depicting the epidermis, dermis, and hypodermis, with a laser pulse applied to a portion thereof.
Figure 2 is a cross-sectional illustration of skin tissue depicting a hair follicle and a sebaceous gland.
Figures 3A and 3B are graphs illustrating the absorption coefficients of human sebum lipid, water, and melanosomes for various wavelengths of light.
Figure 4A is a graph illustrating a surface temperature profile of a target skin area according to a mathematical model of a treatment with a laser pulse.
Figure 4B is a graph illustrating a sebaceous gland temperature profile within a target skin area according to the mathematical model of the laser pulse of Figure 4A.
Figure 5A is a graph illustrating a surface temperature profile of a target skin area before, during, and after a laser pulse treatment with skin cooling, according to a mathematical model.
Figure 5B is a graph illustrating a sebaceous gland temperature profile within a target skin area before, during, and after a laser pulse treatment with skin cooling, according to the mathematical model of Figure 5A.
Figure 5C is more detailed graph illustrating a surface temperature profile for a target skin area during treatment with a laser pulse according to the mathematical model of Figure 5A.
Figure 6A, 6B, and 6C are block diagrams of embodiments of dermatological treatment systems according to the present invention.
Figures 7 and 8 are simplified figures of a handpiece according to an embodiment of the present invention.
Figures 9-16 are flowcharts illustrating treatment methods according to various embodiments of the present invention.
Fig. 17 illustrates a stylistic depiction of a target area for treatment according to various embodiments of the present invention.
Fig. 18 illustrates a stylized block diagram depiction of a system according to embodiments herein.

### Description

Exemplary embodiments of the present disclosure are illustrated in the drawings, which are illustrative rather than restrictive. No limitation on the scope of the technology, or on the claims that follow, is to be implied or inferred from the examples shown in the drawings and discussed herein.

Treatment of many dermatological conditions involve using laser light to heat a target skin area to thermally damage a selected structure within the target skin area. Laser treatment may be ablative or non-ablative, and may result in a healing response to the damaged area to improve the patient's condition. Consistently accurate delivery of power to targeted structures to achieve a desired level of damage to a target structure, while minimizing the delivery of power and corresponding damage to non-targeted structures, has remained an unrealized goal. Although disclosures herein are described in terms of power delivered to targeted structures or absorbed by structures, those skilled in the art having benefit of the present disclosure would appreciate that delivery of energy to targeted structures or energy absorbed by structures, may also be performed and still remain withing the spirit and scope of embodiments herein. The present disclosure is directed to providing systems and methods to achieve these objectives.

As used herein "target skin area" refers to the skin area (sometimes referred to as a "spot") receiving the power of a laser pulse. The target skin area may include the surface skin area illuminated by the laser pulse, as well as deeper structures beneath the surface skin area that receive a portion of the power from the laser pulse. As such, "target skin areas" treated by a laser pulse may refer to a volume of skin as opposed to a true area of an outer surface of the epidermis. A target skin area (i.e., the area "targeted" by the laser pulse and receiving its energy) includes both primary or desired target portions (e.g., a sebaceous gland intended to be thermally damaged to reduce the production of sebum), and collateral or undesired target portions (i.e., overlying tissue for which heating is not desired but cannot be avoided when the primary target is treated). Thermal injury to collateral portions of a target skin area constitute collateral and undesired damage that may be avoided or minimized by systems and methods of the present disclosure.

As used herein, "surface temperature" in reference to a target skin area refers to the temperature of the target skin area as determined or measured at or above the surface of the skin. In particular, where infrared power radiated from a target skin area is used to measure the temperature of the skin surface, the surface temperature determination includes power radiated from deeper in the epidermis in addition to the outermost layer of cells. Without being bound by theory, the strong scattering effects of infrared (IR) wavelengths within the epidermis limit the power emitted and detected to the epidermis's upper 100 microns, and primarily the upper portions thereof. Consequently, "determining a surface temperature" based on detection of radiated infrared power refers to the determination of a composite or average temperature of the upper portions (e.g., tens of microns in depth) of the epidermis, and not merely the outermost layer of skin cells. In embodiments of the present invention, it provides a reliable and precise determination of the temperature of the uppermost portion of the epidermis.

In one aspect, the present invention comprises systems and methods for improved temperature control of a target skin area during the delivery of one or more laser pulses in the treatment of a medical condition. In some embodiments, the present invention comprises systems and methods for control of the temperature of the surface and upper portions of a target skin area of a patient during a laser pulse in the treatment of a dermatological condition. In a particular embodiment, the invention comprises providing a selected cooling power to a first skin area that includes one or more target skin areas. This may include providing a selected cooling power to laser power ratio in treating the target skin areas. In some embodiments, the present invention provides improved temperature control of a target non-surface (*i.e*., deeper) structure in a target skin area during a laser pulse. The invention provides systems and methods with improved efficacy, safety and/or comfort to patients being treated for a range of dermatological conditions.

In one aspect, the invention provides systems and methods of controlling a temperature of a target skin area during a laser pulse that avoid excessive damage to a target structure within the target skin area, and/or undesired damage to overlying non-targeted collateral structures by providing a desired cooling power to a first skin area including one or more target skin areas. In another aspect, the invention provides systems and methods of controlling a temperature of a target skin area during a laser pulse to avoid underheating a target structure, resulting in too little damage to the target structure.

In one aspect, the present invention discloses systems and methods for minimizing the temperature increase of non-target collateral structures overlying a target structure within a target skin area during the delivery of a laser treatment to raise the target structure from a first temperature to a second temperature, such as a damage threshold temperature for a target structure in a dermis. In one embodiment, the laser treatment comprises a single pulse, and the temperature of the target skin area is measured one or more times during the delivery of the pulse.

In one embodiment, the laser treatment comprises a plurality of pulses made during a single heating episode of a target skin area, and the temperature of the target skin area is determined one or more times during the single heating episode. As used herein, a "single heating episode" involves a plurality of pulses where each pulse raises the temperature of the target skin area from a first or baseline temperature immediately prior to the delivery of the pulse, and each successive pulse in the heating episode is applied before the target skin area returns to the first or baseline temperature existing before the immediately preceding pulse, resulting in a sawtooth increase in temperature for a given location within the target skin area. By providing a system having a selected cooling power, it is possible to provide a system with significantly reduced damage to collateral overlying structures. Where a plurality of pulses are used to heat the target skin area to a desired temperature in a single heating episode (*e.g*., a damage threshold temperature for a target structure such as a sebaceous gland or a sweat gland), the temperature of the target skin area may be determined during a pulse, between pulses, or a combination of during and between pulses of the single heating episode.

In one embodiment, the invention comprises a method of determining the length of a laser treatment pulse based on determining the surface temperature of a target skin area one or more times during the delivery of the laser treatment pulse. By determining the surface temperature of the target treatment area during the delivery of the laser treatment pulse, the laser pulse may be terminated when the skin reaches a desired temperature that avoids overheating the target skin area and causing excessive damage to non-targeted structures, or terminating the pulse too early, with too little damage to the target structure(s). In a preferred embodiment, the invention may also include skin cooling (*e.g*., contact cooling applied to the skin surface) using a contact cooling element having a selected cooling power relative to the laser power to enable heating of deeper structures (*e.g*., a sebaceous gland, a hair follicle root, or a sweat gland) to a damage threshold temperature while minimizing thermal damage to overlying collateral tissue structures. In one embodiment, the ratio of the cooling power and the laser source power is at least 20%. In an alternative embodiment, the ratio of the cooling power and the laser source power is at least 15%. In one embodiment, the ratio is no greater than 50%. In another embodiment, the ratio is no greater than 40%.

As used in connection with temperature determinations, "real-time" refers to temperature determinations (*e.g*., temperature measurements or calculations based on data from a temperature sensor) performed during an action (*e.g*., during the cooling of a target skin area or the delivery of a laser pulse to a target skin area) and used by a processor to determine a timepoint for terminating the action or initiating another action. In one aspect, the invention comprises real-time temperature determinations during the cooling of a first skin area, and the use of the temperature determinations to perform an action such as terminating the cooling process, initiating delivery of a laser pulse to a target skin area, or terminating delivery of a laser pulse to a target skin area.

In one aspect, the invention comprises real-time temperature determinations performed during the delivery of a therapeutic laser pulse (or during or between delivery of a plurality of pulses comprising a single heating episode), which may be used (*e.g*., by a processor executing a treatment algorithm) to perform an action such as initiating or terminating delivery of a laser pulse, adjusting a parameter of a laser pulse, or initiating, terminating, or adjusting (*e.g*., increasing or decreasing the power of) a cooling process associated with the delivery of therapeutic laser pulse(s). In one embodiment, the ratio of the cooling power and the laser source power is at least 20%, while in an alternative embodiment, this ratio is at least 15%. In one embodiment, the invention may comprise changing the cooling-to-heating power ratio from a first ratio (e.g., 15%, 20%, 25%), to a second ratio, which may be greater or lesser than the first ratio.

In one aspect, the invention comprises a method of treating a patient having hyperhidrosis (*i.e*., excessive sweating) by controlled heating of a target skin area from a first surface temperature to a second surface temperature, where the second surface temperature corresponds to a temperature resulting in thermal damage to a sweat gland within the target skin area. In one embodiment, the method comprises cooling a first skin area comprising one or more sweat glands using a contact cooling element prior to application of one or more laser pulses to a target skin area within the first skin areas, wherein the contact cooling element comprises a cooling power that is at least 15%, more preferably 20% or more, of the power of the laser source generating the laser pulses. In one embodiment, the laser treatment pulse is terminated when the second surface temperature reaches a value indicative of the deeper sweat gland reaching a sweat gland treatment temperature.

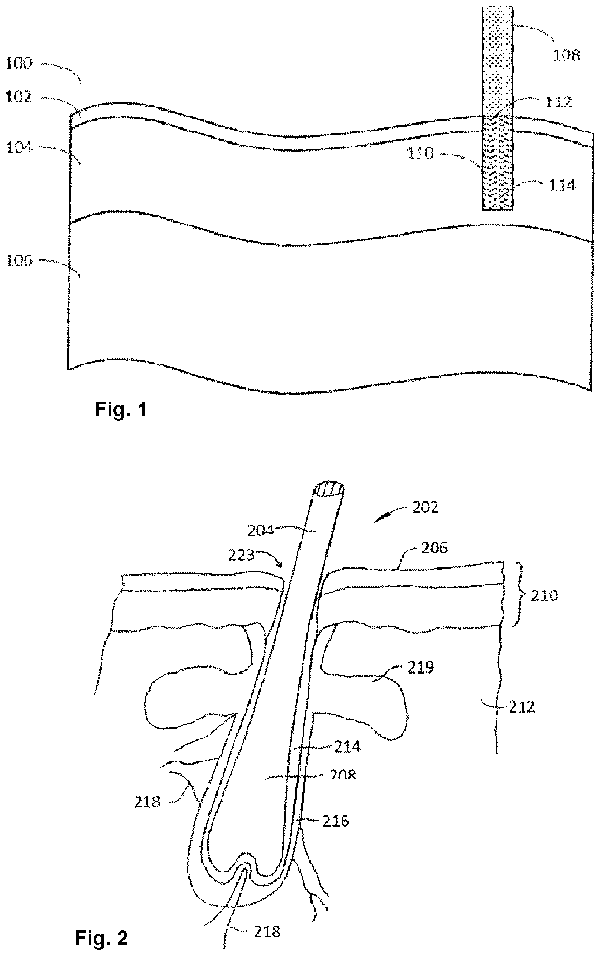

Figure 1 is a side view illustrating a cross-sectional view of a portion 100 of the skin of a patient, including the outermost epidermis 102, the middle layer or dermis 104, and the bottom layer or hypodermis 106. The epidermis 102 has a thickness of about 80-100 µm, which may vary from patient to patient, and even for a single patient depending upon age, health status, and other factors. It includes up to five sub-layers (not shown) and acts as an outer barrier.

**The** dermis 104 has a thickness of about 1-5 mm (1000-5000 µm). It contains the blood vessels, nerves, hair follicles, collagen and sweat glands within the skin. Because skin conditions frequently involve structures in the dermis, many laser systems must include sufficient power to penetrate into the dermis to reach and treat structures therein. Careful selection of a number of parameters must be made in the design and construction of laser systems for treatment of a variety of skin conditions to achieve a desired level of damage to a target structure while minimizing or avoiding damage to non-targeted (*e.g*., overlying) structures. For example, incorrect selection of the laser wavelength, pulse width, power per pulse, the use (or nonuse) of a seed laser, or the pump power of the laser source or amplifier may result in undesired damage as well as poor performance in treating a dermal structure of interest. Numerous other system choices, such as the use or non-use of an articulating arm for delivery of the laser light to a handpiece for application to the patient's skin, may also affect overall system performance.

The lowest layer of the skin is the hypodermis 106, which includes adipose tissue and collagen. The hypodermis 106 helps control body temperature by insulating the structures of the body below the skin. In addition, the hypodermis protects the inner body tissues from damage by absorbing shock and impacts from outside the body. Because the hypodermis contains fat, its thickness varies widely from person to person based on diet, genetic makeup, and other factors.

Figure 1 depicts a laser beam 108 applied to a target skin area 110 of the skin 100. The target skin area 110 comprises a surface skin area 112, as well as underlying skin tissue 114 that absorbs at least a portion of the power of the laser beam 108.

Figure 2 is a side view of the skin of a patient illustrating in simplified form, a hair 202 including a hair shaft 204 extending beyond the exterior skin surface 206. Hair shaft 204 includes a root 208 located below epidermis 210 in the dermis 212. The base, or papilla, of root 208 is located about 4 mm below exterior skin surface 206. Root 208 is housed within hair follicle 214 and is surrounded by tissues including connective tissue sheath 216 and blood vessels 218. Follicle 214 includes a sebaceous gland 219 below an opening 223. Sebaceous glands such as gland 219 are typically located at depths ranging from about 0.3 mm (300 µm) to about 2.0 mm (2000 µm) below exterior skin surface 206, but their depth varies depending upon body location.

Epidermis 210 includes melanin (not shown), a dark pigment found in tissues of the hair, skin and eyes. Melanin, the primary determinant of skin color, is located within globular structures known as melanosomes, which are produced by skin cells called melanocytes. Darker skin has more melanosomes (and thus more melanin) per unit skin area compared to lighter skin. Laser systems targeting deeper structures such as sebaceous gland 219 in the dermis may present a higher risk of patient discomfort where wavelengths having a relatively high absorption coefficient in melanin are used. Without being bound by theory, when laser light at wavelengths readily absorbed by melanin is applied to dark skin (or dark tattoos having ink particles that absorb laser light at similar wavelengths to melanin), the power absorbed by the melanin (or tattoo ink particles) attenuates part of the laser power that otherwise would reach deeper structures absent the melanin or ink particles, heating the skin of the epidermis and/or upper dermis to a greater degree than lighter/un-tattooed skin.

Accordingly, in one aspect, the present invention provides laser treatment systems to minimize discomfort by adjusting one or more parameters based on the skin type of the patient. In one embodiment, the invention provides systems and methods for determining a skin type of a patient and automatically adjusting one or more treatment parameters based on the skin type of the patient. This may involve, for patients having darker skin types, one or more of: increasing a cooling power to laser source power ratio above a ratio applicable for patients with lighter skin types; selecting a cooling power that is at least 20%, and preferably at least 25% of the laser source power; lowering a first skin temperature at which a laser pulse is initiated and applied to the patient's skin; lowering a fluence of a laser pulse; lowering a peak power of a laser pulse; providing a laser pulse having a longer pulse width; and providing a larger beam diameter for a pulsed laser therapy. In one embodiment, the ratio of the cooling power and the laser source power is at least 20%, while in an alternative embodiment, this ratio is at least 15%.

Successful treatment of acne involves damaging sebocytes and/or sebaceous glands. This involves heating sebum, which is produced by and located within the sebaceous glands, to damage the gland. Accordingly, it is desirable to select a wavelength of light that is highly absorbed by sebum, preferably more so than competing skin chromophores (*e.g*., water), to limit the damage to non-targeted tissue and concentrate the laser power delivered into the targeted sebaceous gland to the exclusion of non-targeted tissues and structures. In addition, because sebaceous glands are relatively deep structures located in the dermis at depths of 300-2000 µm (0.3-2.0 mm), it is desirable to select a wavelength of light capable of non-ablative penetration to these depths.

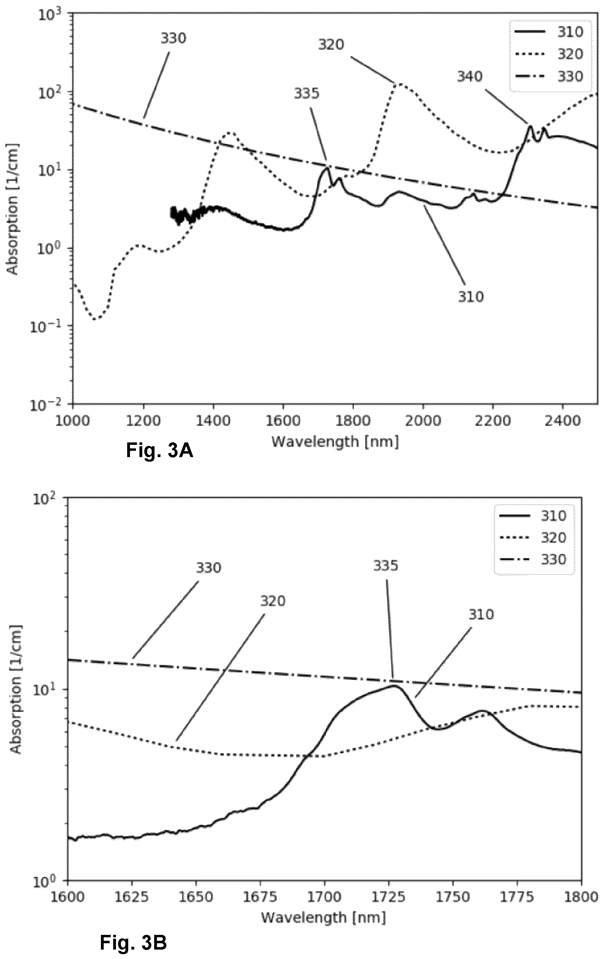

Figures 3A and 3B are graphs illustrating the absorption curves for several chromophores of interest (water, sebum, and melanosomes) at wavelengths of light for portions of the near-infrared spectrum (about 750 nm - 1400 nm) and the short-wavelength infrared spectrum (about 1400-3000 nm). Figure 3A illustrates the absorption curve 310 for sebum, the water absorption curve 320, and the absorption curve 330 for melanosomes. Figure 3A demonstrates that the sebum absorption curve 310 has a peak at about 1727.5 nm, meaning that sebum absorbs laser light at this wavelength more strongly than light at other nearby wavelengths, e.g., 1650 nm or 1800 nm. The absorption coefficient of water is less than that of sebum in a range of from about 1693 nm to about 1742 nm, and within a range of from about 2280-2360 nm.

As shown more clearly in Figure 3B, the absorption coefficient for sebum at a peak of about 1727.5 nm is approximately twice that of water, and is only slightly less than that of melanosomes. Accordingly, in one embodiment, the invention comprises a laser providing pulsed laser light at a wavelength of between 1693-1742 nm, more preferably at about 1720-1730 nm, and more preferably still at about 1727.5 nm.

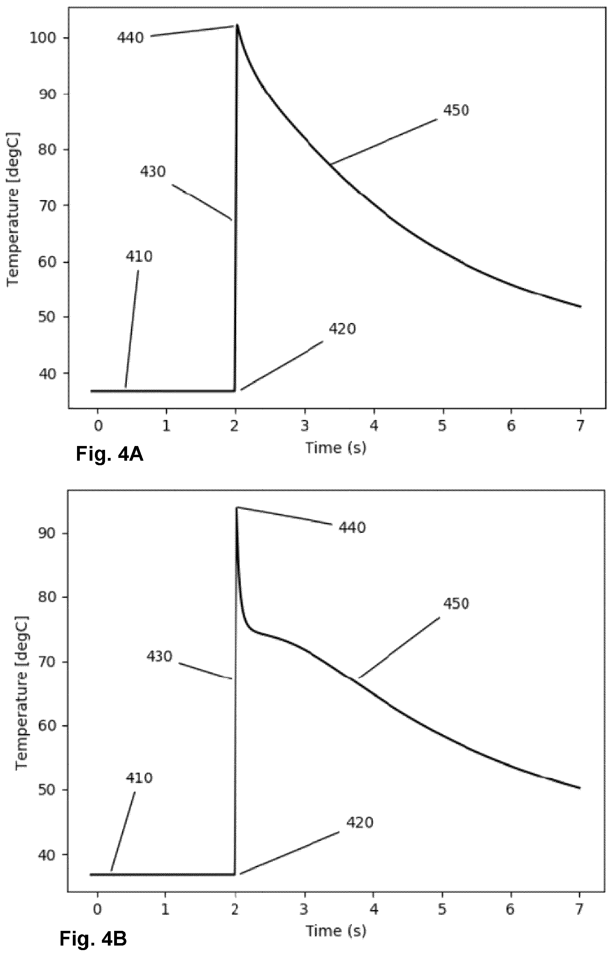

Figures 4A and 4B illustrate exemplary temperature profiles of the surface of a target skin area (Figure 4A) and a sebaceous gland located below the skin surface (Figure 4B) during a laser pulse according to a mathematical model of one embodiment of the present invention. The laser pulse is intended to raise the temperature of the sebaceous gland to result in a desired cell population death for sebocytes receiving laser pulse energy. In this embodiment, the laser pulse is a tophat pulse (*i.e*., having a uniform intensity profile over the covered area) used to heat a target skin area, with a wavelength of 1727.5 nm, a pulse duration of 30 msec, a beam diameter of 2.8 mm, a power of 75 W, a pulse energy of 2.25 J, and a fluence of 37 J/cm2. For illustration, the skin is shown remaining at body temperature for 2 seconds prior to the application of the pulse, although the initial time period before pulse initiation could be shown as any time period.

Referring to Figure 4A, at time t= 2 seconds, a single pulse of laser light having the parameters noted above is initiated and applied to a target skin area, depicted at point 420. The surface temperature of the skin rises as shown by line 430, to slightly above 100°C (peak 440). After pulse termination, the skin surface cools rapidly (curve 450), falling to below 60°C within 4 seconds (t=6 seconds) after pulse termination.

Figure 4B illustrates the temperature profile of a sebaceous gland at a depth of 650 µm below the skin surface in the laser pulse model of Figure 4A. The skin remains at body temperature for 2 seconds (410) prior to the initiation of a pulse (421). The temperature of the gland rises (430) to a maximum 440 of about 92°C-less than the skin surface temperature in Figure 4A due to scattering and absorption by the tissue overlying the sebaceous gland. Because the pulse wavelength of 1727.5 nm is preferentially absorbed by the sebaceous gland (as discussed in connection with Figures 3A and 3B), comparatively more power from the laser pulse is absorbed by the oily tissue compared to overlying tissue containing higher water content. Consequently, the temperature profile (450) of the sebaceous gland after pulse termination differs significantly from that of the skin surface depicted in Figure 4A. Although initially falling rapidly to about 85°C, the temperature thereafter falls more slowly than the surface temperature shown in Figure 4A.

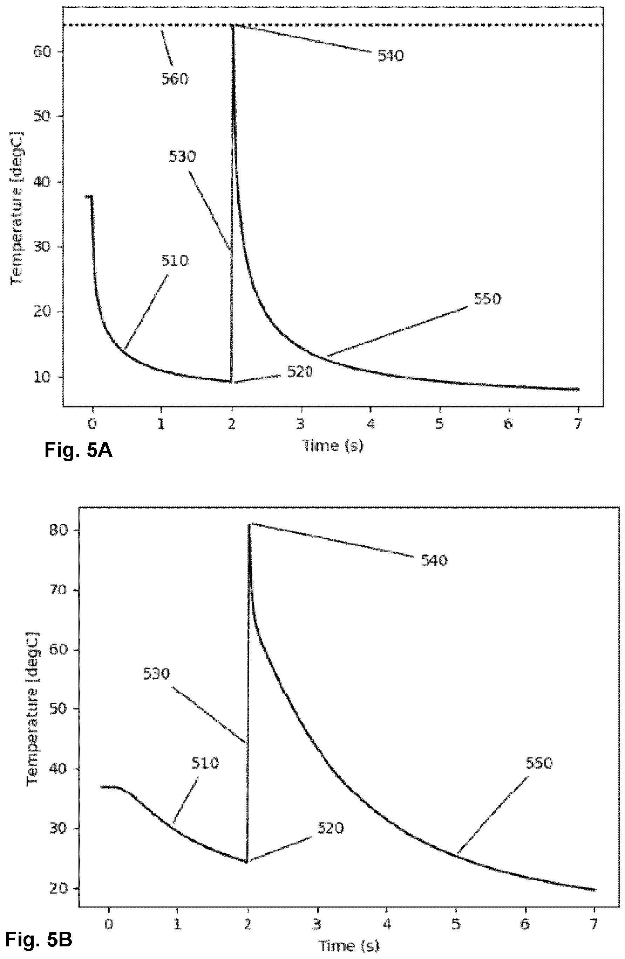

The pulse in Figures 4A and 4B has power levels below those necessary to ablate skin tissue, but are likely to result in significant discomfort when they persist for, *e.g*., four seconds or longer. Accordingly, the pulse depicted in Figure 4A would have limited application as a viable treatment. In one embodiment, temperatures may be lowered by skin cooling, as described in connection with Figures 5A and 5B.

Figures 5A and 5B illustrate exemplary temperature profiles of portions of a target skin area during a laser pulse according to a mathematical model of another embodiment, with the laser pulse having the same parameters as those of Figures 4A and 4B (wavelength λ=1727.5 nm; pulse duration = 30 msec; beam diameter = 2.8 mm, power = 75 W; pulse energy = 2.25 J; fluence = 37 J/cm2). However, in contrast Figures 4A and 4B, in Figures 5A and 5B the target skin area is cooled prior to, during, and after the application of the laser pulse.

Persons of skill in the art will appreciate that many known methods and modes of precooling skin may be used. The embodiment of Figures 5A and 5B are modeled on a system having a contact cooling element applied to a first skin area that includes a target skin area to be treated by the laser pulse. The contact cooling element includes a cooling window that, in some embodiments, directly contacts the first skin area, and the target skin area irradiated by the laser pulse is wholly located within the first skin area. Although a variety of materials may be used as the contact cooling window, in the embodiment of Figures 5A and 5B, the cooling system includes a sapphire cooling window cooled by a thermoelectrical cooler (TEC). The sapphire cooling window has a thickness of 3 mm and a diameter of 1 inch (25.4 mm), although many different sizes, shapes, thicknesses, and materials may be used in cooling window embodiments disclosed herein. For example, although the cooling window modeled in the embodiment of Figures 5A and 5B was circular, other cooling window shapes such as square, rectangular, or other polygonal or nonpolygonal shapes could be used in different embodiments. The cooling window was modeled as cooled to 5°C.

In alternative embodiments, non-contact cooling systems (*e.g*., cold air or other fluid circulated onto or across the surface of a target skin area, or sprayed evaporative coolants) may be used to cool the skin. Without being bound by theory, it is believed that the thermal resistivity of the skin and the thermal coupling between the skin and gases such as air typically preclude non-contact systems from providing adequate cooling capacity during the delivery of laser pulses to effectively treat deeper target structures and prevent the skin surface from reaching temperatures likely to cause significant discomfort. Accordingly, contact cooling systems are preferred.

Referring to Figure 5A, the contact cooling element at 5°C is applied to the skin at time t=0. The temperature falls rapidly along curve 510 to a target temperature of about 10°C at time t=2 second, at which point (520) the laser pulse is applied to the skin. Delivery of the laser pulse is continued until a target surface temperature 560 of the target skin area is reached at point 540, when the laser pulse is terminated. Because the contact cooling element continues to cool the skin by direct contact during and after the laser pulse, the surface temperature falls rapidly along curve 550 after the laser pulse is terminated. The cooling element may be configured to provide a cooling power such that the ratio of the cooling power and the laser source power is at least 20%, while in an alternative embodiment, this ratio may be at least 15%

Figure 5B illustrates the temperature profile of a sebaceous gland located at a depth of 650 µm below the skin surface in the cooling/pulse delivery process of figure 5A. When the contact cooling element is applied to the skin at time t=0, the gland temperature declines as shown by curve 510, but much less rapidly than the surface temperature decline depicted in Figure 5A. The laser pulse is initiated at point 520, and the temperature of the gland rises along line 530 until terminated at point 540. The gland temperature thereafter falls along line 550, but the curve is noticeably less steep (*i.e*., the temperature fall is less rapid) than the surface temperature decline following the pulse termination.

Because direct measurement of the gland temperature is difficult or impossible given its depth, in embodiments of the present invention, surface skin temperature may be monitored as an indirect indication of the gland temperature. Because the goal of the laser treatment is to heat the sebaceous gland to a damage threshold temperature, the cooling of the gland (as opposed to the skin surface) shown by curve 510 in Figure 5B is undesired, but tolerated as an unavoidable consequence of the precooling of the overlying collateral skin tissue. By precooling the overlying skin to a desired surface temperature of about 10°C (Figure 5A), a downward cooling wave is generated in the target skin area, propagating from the skin surface toward the deeper tissues in the dermis and hypodermis. This precooling process may be controlled such that, for a sebaceous gland within a known depth range, when the laser pulse is delivered to heat the target skin area, the protectively cooled overlying skin remains below a damage threshold temperature while the target sebaceous gland reaches a damage threshold temperature. This is facilitated by selecting a laser wavelength at which the absorption coefficient of sebum/sebaceous gland tissue exceeds that of water, the primary chromophore in the overlying dermal and epidermal tissue.

Comparing Figures 5A and 5B, the precooling process allows the sebaceous gland to reach a temperature of about 78°C at the termination of the laser pulse-about 13°C above the target skin area at the surface (about 62°C) at pulse termination. Sometimes, the precooling process may be configured to allow the sebaceous gland to reach a temperature about 16°C above that of the surface at pulse termination. Although the overlying collateral tissue is unavoidably heated during pulse delivery, careful precooling of the overlying tissue before initiating the laser pulse allows the surface temperature to be precooled to a temperature well below that of the sebaceous gland when the laser pulse is initiated (about 10°C for the surface vs. about 22°C for the sebaceous gland as shown by Figures 5A and 5B at point 520). This temperature difference occurs because the cooling window causes a thermal gradient between the skin surface and deeper structures. In addition, the pulse wavelength (1727.5 nm) is more highly absorbed by the sebaceous gland than the non-target or collateral overlying tissues. As a result, the non-targeted tissue overlying the sebaceous gland is heated to a lower temperature (about 63°C, Figure 5A, point 540) than the targeted sebaceous gland (about 81°C, Figure 5B, point 540), minimizing damage to the non-targeted tissue and patient discomfort.

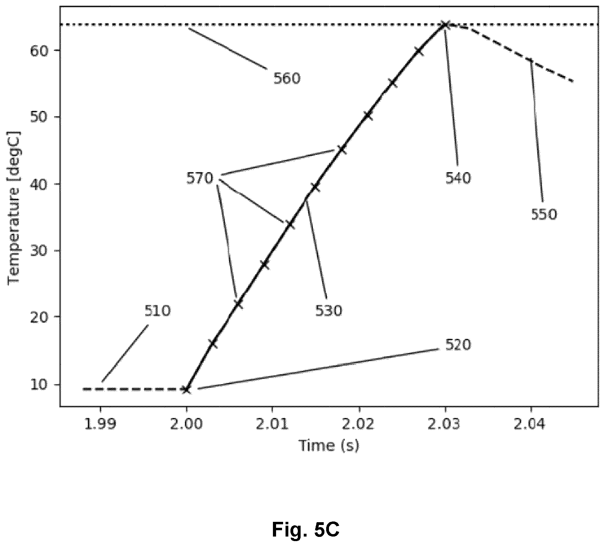

Certain systems and methods of the present invention are facilitated by a method of controlling the duration of a pulse to limit the surface temperature of a target skin area. Figure 5C demonstrates a method of achieving such control by monitoring the skin temperature during pulse delivery. The surface temperature of the skin may be determined one or more times during pulse delivery, and the pulse may be terminated based on skin temperature(s) thus determined. In one embodiment, the skin temperature is periodically determined during the pulse delivery, and the pulse is terminated when the surface skin temperature reaches (or nears) a threshold temperature.

Figure 5C, illustrates a more precise temperature profile of the delivery of the pulse of Figures 5A and 5B. From time t=1.99 to t=2.00 seconds, the temperature of the skin (modeled in Figure 5C at a depth of 100 µm) is about 10 °C (line 510). At time t=2.00 seconds (520), the pulse is initiated and applied to the skin through the sapphire cooling window. Simultaneously, the first of a plurality of surface temperature determinations (570) is made. Pulse delivery continues (line 530), and the surface temperature rises until pulse termination (540). After termination, the surface temperature falls (line 550). During pulse delivery, multiple temperature determinations 570 are made at equal intervals, although the frequency of temperature sampling may vary based on a variety of factors, time frame desired for heating the tissue, thermal relaxation of the target structure, pulse fluence, pulse power, pulse wavelength, and exogenous factors such as the damage threshold for the particular target structure (*e.g*., a sebaceous gland, hemoglobin, melanin, *etc.*), and other factors. A desired temperature sampling interval, *e.g.,* 100 msec or less (*i.e*., performing 10 or more temperature determinations per second) may be used. The skin surface temperature may be determined at a sampling interval or time between temperature determinations of 0.001-100.0 msec (*i.e*., 1-100,000 µsec, 10 to 1 million temperature determinations/second).

In Figure 5C, the skin temperature determinations are made by sensing infrared radiation from the surface of the target skin area, although other known methods may be used. Although Figure 5C depicts a constant sampling interval, non-constant intervals, *e.g*., varying based on the difference between a measured temperature and a desired threshold, or on other exogenous factors such as the operating speed of the sensor or processor, may be used. In one embodiment, the sampling interval is increased as the surface temperature approaches a threshold temperature.

In one embodiment, the laser pulse may be terminated after the first temperature determination at or above the temperature threshold. In another embodiment, the pulse may be terminated based on a predicted timepoint at which the skin surface will reach the threshold temperature, without requiring that the temperature be reached or exceeded. For example, periodic predictions of when the threshold temperature will be reached may be made, *e.g*., by fitting a straight line or polynomial function to the temperature data each time a subsequent temperature determination is made, and projecting the function forward to determine a prediction of when the temperature threshold will be reached.

Figures 5A and 5B illustrate methods of treating a sebaceous gland according to one embodiment of the present invention. It will be appreciated, however, that embodiments of the present invention may be used to treat other structures in the dermis or hypodermis (*e.g*., sweat glands, hair follicles, *etc*.) by facilitating precise control of surface and deeper temperatures within a target skin area.

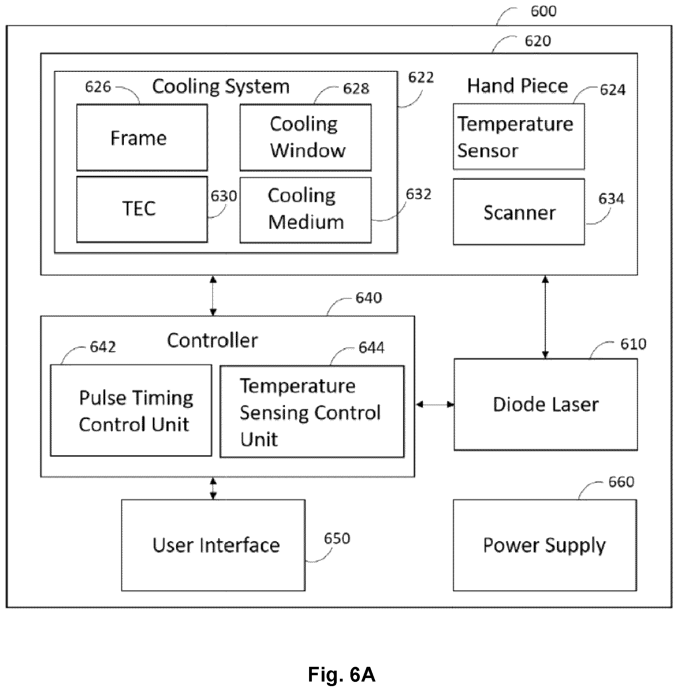

Figure 6A is a schematic illustration, in block diagram form, of an embodiment of a therapeutic laser system 600 for providing therapeutic laser pulses as described in connection with Figures 5A-C. A diode laser 610 generates light at a wavelength with a high absorption coefficient in a target tissue such as sebaceous gland tissue, although other tissue types may be targeted. Diode laser 610 is optically coupled, e.g., by an optical fiber, articulating arm, or other optical coupling elements known in the art, to a handpiece 620 for delivery of a laser pulse to a target skin area. Although diode laser 610 is used in Figure 6A, other laser types (*e.g.*, fiber lasers, dye lasers, *etc.)* may be used in different embodiments.

Handpiece 620 includes a cooling system 622 for cooling a first skin area that includes a target skin area within the first skin area. Cooling system 622 includes a contact cooling element comprising a cooling window 628 maintained in a fixed position in contact with a heatsink portion of a thermoelectric cooler (TEC) 630 by a window frame 626. Cooling window 628 may comprise any of a variety of IR-transmissive materials, including sapphire, ZnS, diamond, ZnSe, and other thermally conductive materials transmissive to infrared light. In alternative embodiments (not shown), the contact cooling element may comprise structures in addition to cooling window 628, such as a copper (or other high thermal conductivity materials) cooling element that is not light-transmissive to provide additional cooling capacity.

TEC 630 may be a Peltier-type cooler having a warm side and a cold side (not shown). The heatsink portion of the TEC 630 is part of the cold side, which removes heat from the cooling window 628 to maintain the window at a desired temperature as it contacts the first skin area. A cooling medium 632 removes heat from the hot side of the TEC 630 to prevent heat buildup in handpiece 620. The cooling medium may comprise circulating cold water, although other thermally conductive fluids may be used in different embodiments.

To ensure efficient skin cooling, it is necessary to maintain good contact between the skin and cooling window 628 during treatment. In one embodiment (not shown), one or more contact sensing elements may be provided to detect when the cooling window 628 is in proper contact with the first skin area. The contact sensing element(s) may be coupled to, or separate from, cooling window 628 and/or frame 626, and may comprise, *e.g*., one or more electrical contacts capable of sensing electrical activity, conductivity, or resistance of the skin indicative of adequate skin/cooling window contact. Other contact sensing elements (*e.g*., ultrasonic sensors) detecting different skin parameters or features associated with proper contact (*e.g*., force, vibration, pressure, temperature, the presence of sweat or skin oils) may also be used. Contact sensing systems suitable for use in the present invention are disclosed in related US Patent App. Ser. No. 17/551,135, filed December 14, 2021, which is hereby incorporated in its entirety.

One or more skin contact indicators (not shown) may alert a user to the contact status between the skin and cooling window 628. The skin contact indicator may indicate when the contact element(s) are-or are not-in good contact with the first skin area and may prompt the user to manipulate the handpiece to restore good contact when necessary. The skin contact indicator(s) may comprise, *e.g*., an LED indicator on handpiece 620 that displays a first color when good skin contact exists and a second color when the window 628 is not in proper contact with the skin. Other indicators, such as an audible sound or alarm, may also be provided, and the system may be interlocked such that the system will not apply (or will terminate) a laser pulse if good contact between the cooling window 268 and the skin is absent.

In some embodiments, handpiece 620 includes a temperature sensor 624 for sensing a surface temperature of the target skin area. Temperature sensor 624 may sense the target skin area temperature one or more times before pulse delivery (*e.g*., during a precooling step), during pulse delivery, or after pulse delivery (*e.g*., during a postcooling step). During delivery of a pulse to a target skin area, the surface temperature may be influenced by two different heating mechanisms, including power absorbed directly from the laser, and thermal bloom resulting from energy conducted from deeper skin tissue as the thermal energy absorbed by deeper structures relaxes into the environment. Thermal bloom from deeper structures back to the skin surface may be a significant cause of epidermal damage in laser systems targeting relatively deep structures such as sebaceous or sweat glands. Therapeutic laser systems such as system 600 enable improved treatment outcomes by ensuring that the surface temperature of a target skin area remains below a desired surface temperature even while heating deeper structures to higher temperatures, minimizing skin damage and patient discomfort.

Temperature sensor 624 may sense the surface temperature of the target skin area one or more times during the delivery of a laser pulse from diode laser 610. Temperature sensor 624 may be capable of sensing the surface temperature of the target skin area at from 10 to 1 million times per second. In one embodiment, temperature sensor 624 comprises an infrared radiation detector to detect infrared energy radiating from the surface of the target skin area through the cooling window 628, and a processor (*e.g*., controller 640 as discussed below) to determine the surface temperature of the target skin during a treatment pulse based on data received from the temperature sensor 624. Other types of temperature sensors 624 may be used.

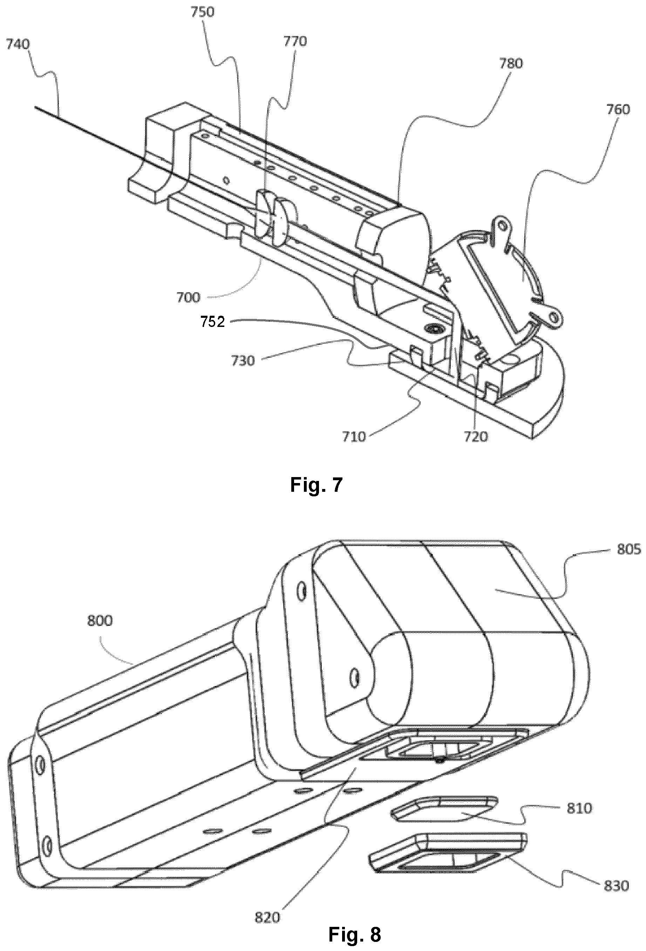

Handpiece 620 also includes a scanner 634 to sequentially direct laser pulses to different target skin areas within a first skin area in contact with cooling window 628. In some embodiments, cooling window 628 may provide contact cooling to a first skin area that is significantly larger than a single target skin area. After a first target skin area is treated by a laser pulse, scanner 634 may be used to redirect subsequent pulses from the diode laser 610 to a new (*i.e*., second, third, *etc.*) target skin area within the larger first skin area cooled by the cooling window 628. When a desired number of target skin areas have been treated at a single cooling window position, the user may reposition the cooling window to a new position covering a new skin area, and a different group of target skin areas within the new skin area may be treated. In one embodiment, scanner 634 may comprise a mirror (see Fig. 8) whose position may be adjusted on two or more axes, *e.g.,* by motor(s), to direct successive laser pulses to different target skin areas within the cooling window, enabling treatment of a relatively high proportion of the first skin area in contact with the cooling window. In alternative embodiments, scanner 634 may be omitted.

The system 600 further includes a controller 640, which may comprise one or more processing elements such as microprocessors, microcontrollers, field programmable gate arrays (FPGAs), etc. to control the operations of the system. Controller 640 includes a pulse timing control unit 642 that controls the timing of the laser pulses from diode laser 610, including initiating the pulse at a first timepoint and terminating the pulse at a second timepoint. The pulse timing control unit 642 may receive data from temperature sensor 624, and may initiate the therapeutic laser pulse at a first timepoint based on, *e.g*., a determination that the surface temperature of the target skin area has been cooled to a desired temperature (*e.g*., a specific below body temperature such as 15°C, 10°C, 5°C, 0°C, -5°C, -10°C, etc.). Alternatively, pulse timing control unit 642 may control the delivery of the pulses based on predetermined time intervals (e.g., a user-selected pulse width, pulse frequency, etc.). Controller 640 may control the cooling process such that the ratio of the cooling power and the laser source power is at least 20% during the laser treatment (*e.g*., during precooling, laser pulse delivery, and postcooling steps). In an alternative embodiment, the ratio of the cooling power and the laser source power is at least 15%. Pulse timing control unit 642 may also terminate the therapeutic laser pulse at a second timepoint based on, *e.g*., a predetermined pulse width, or a determination that the surface temperature of the target skin area has reached a threshold temperature (*e.g.*, a surface temperature indicating that a deeper target structure such as a sebaceous gland has reached a damage threshold such as a temperature in the range of 60°C - 75°C).

Controller 640 may also include a temperature sensing control unit 644 that controls the operation of the temperature sensor 624. In particular, temperature sensing control unit 644 ensures that the surface temperature of a target skin area is determined or measured at a desired (*e.g*., programmed or predetermined) sampling rate such as 10 or more times per second. Controller 640 may synchronize the operations of the temperature sensing control unit 644 with the pulse timing control unit 642. In some embodiments, temperature sensing control unit 344 may be omitted.

In various embodiments, the pulse timing control unit 642 and the temperature sensing control unit 644 may comprise one or more of software, firmware, or other programming code operating in the controller 640. In one embodiment, the pulse timing control unit 642 and the temperature sensing control unit 644 may comprise separate processors or sub-processors within controller 640. A wide variety of hardware and software designs may be used to achieve the functions described herein, and all are considered as within the scope of the present disclosure.

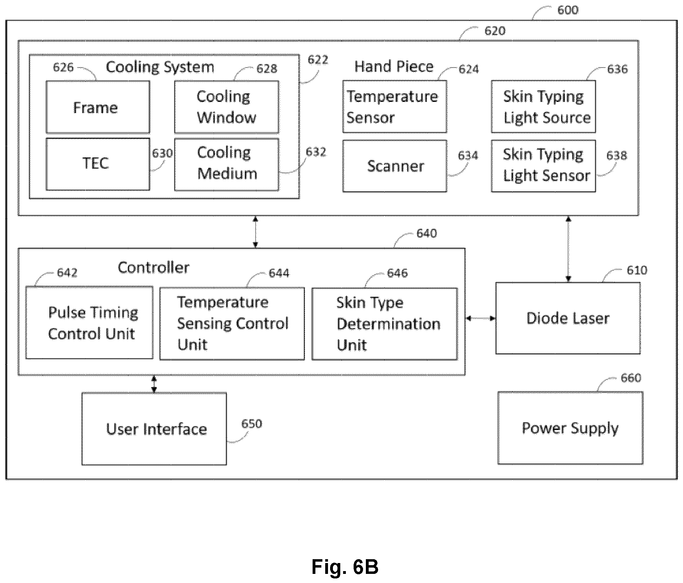

Controller 640 may also control other operations within the therapeutic laser treatment system 600 (*e.g*., software and firmware units and subunits, timers, mechanical or electrical elements or subsystems, etc.). These functions may also include, without limitation, control of the positioning of scanner 634 and thus the location within the cooling window 628 of the target skin areas to receive laser pulses. Controller 640 also controls the operation of cooling system 622, including without limitation the temperature at which the cooling window is maintained (which may be determined by a user or by the patient's skin type as described in connection with Figure 6B), the cooling capacity (*i.e*., the thermal power removal rate of the TEC), status alarms, etc.

A user interface 650 allows a system user to select or program one or more parameters (*e.g*., beam diameter or spot size, fluence, wavelength, target temperature of the surface of the target skin area, cooling temperature of the target skin area at which a pulse may be delivered, cooling system power, *etc*.) to direct the operation of the therapeutic laser system 600. User interface 650 also displays various status indicators and data to the user associated with the system and/or a treatment session, such as the current laser parameters, duration of treatment, number of pulses delivered, cooling to laser power ratio, *etc.* Controller 640 may also receive and process inputs from the user interface 650, and may provide outputs to the user interface as well.

Finally, the system 600 includes a power supply 660 for providing power to one or more of the foregoing portions of the system. In one embodiment, power supply 660 may comprise a power supply coupled to a standard A/C power outlet to convert AC to DC power at one or more voltages, and may include a battery (*e.g*., for backup in the event of a power outage), a supercapacitor, *etc.* Power supply 660 also provides power to controller 640, which in turn includes a current-controlled power supply for driving the diode laser 610 and/or other system components and subassemblies at rapid switching rates based on inputs from controller 640 (*e.g*., pulse timing control unit 642 or temperature sensing control unit 644), cooling system 622, temperature sensor 624, and scanner 634.

Figure 6B is a schematic illustration of an alternative embodiment of the therapeutic laser system 600 of Figure 6A. The therapeutic laser system of Figure 6B provides a system capable of determining the skin type of a patient, and adjusting one or more treatment parameters based on the skin type, or providing information to a user for making such adjustments. Like numbers are used for like elements in Figures 6A and 6B, and the discussion herein of Figure 6B will omit or limit previously discussed elements of Figure 6A for simplicity, brevity, and to avoid repetition. Elements previously described in connection with Figure 6A will have similar functions in Figure 6B.

The system of Figure 6B allows one or more treatment parameters of the system 600 to be adjusted to minimize discomfort and/or pain to patients that may result from differences in skin type. Handpiece 620 includes a skin typing light source 636 for applying a multi-wavelength light signal to the skin of a patient to allow the system to determine a skin type of the patient. Although different skin typing systems may be used, in one embodiment the system 600 of Figure 6B is adapted to determine a Fitzpatrick skin type of the patient. Skin typing light source 636 may generate noncoherent, multi-wavelength light in visible and/or IR light ranges. A skin typing light sensor 638 is provided to sense a portion of the light from light source 636 reflected from the skin of the patient.

Controller 640 includes a skin type determination unit 646 that receives data from the skin typing light sensor 638 relating to, *e.g*., the absorbance or non-absorbance of the patient's skin of particular wavelengths of light from the skin typing light source 636. The skin type determination unit 646 analyzes the absorbance/non-absorbance data from the skin typing light sensor 638 and determines a skin type of the patient. Controller 640 includes logic (not shown) to modify one or more aspects of the laser treatment based on the patient's skin type, and/or provide data to a user, to maintain the skin surface temperature below a desired maximum temperature during treatment.

Without being bound by theory, patients with darker skin (*i.e*., a higher melanin content than lighter skin) may experience a more rapid temperature rise during the delivery of a laser pulse as relatively more power from the pulse is absorbed by the more highly concentrated melanin particles in the skin. To avoid an excessive temperature (and patient discomfort/pain), controller 640 may, for example, provide additional cooling (*e.g*., driving the TEC at a higher cooling power, using a longer cooling time before laser pulse delivery) for patients with darker skin; lower a target skin temperature at which a therapy pulse is initiated (*e.g*., initiate therapy when the skin is cooled to 5°C for patients with darker skin instead of 10°C for lighter-skin patients); lower a fluence of the therapeutic laser pulses to deliver less power per unit time for darker skin patients; lower a peak power of the laser pulses of a laser therapy for darker skin patients. The controller may also modify or change other parameters (*e.g*., pulse duration, spot size) to ensure efficacious surface temperature control in the treatment of a wide range of skin types.

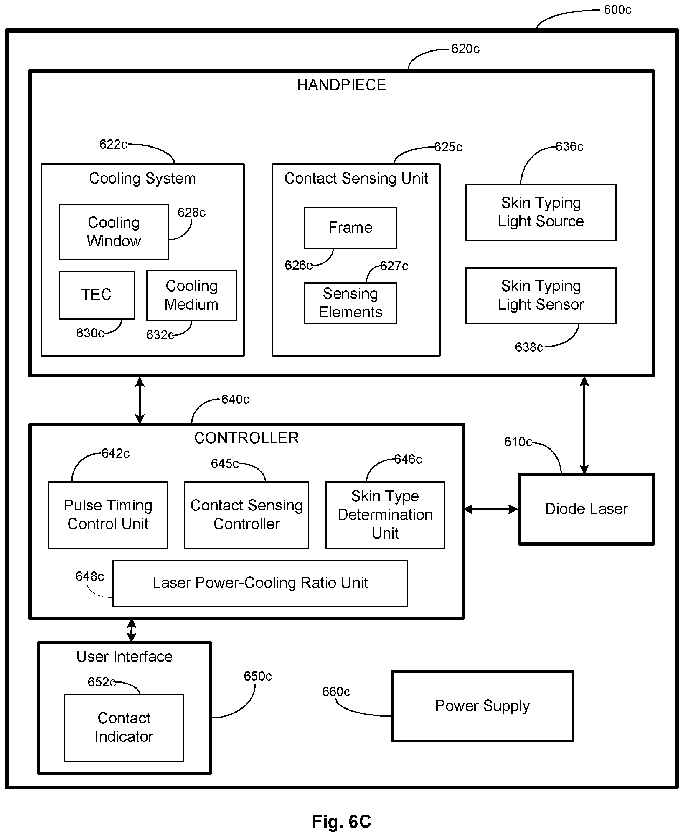

Figure 6C is a schematic illustration of yet an alternative embodiment of the therapeutic laser system 600 of Figure 6A.

Figure 6C is a block diagram of an alternative embodiment of therapeutic laser system 600 of Figure 6A. Like numbers are used for like elements in Figures 6A, 6B, and 6C, and the discussion of Figure 6C omits or limits previously discussed elements of Figure 6A for brevity and to avoid repetition. Elements previously described in connection with Figure 6A will have similar functions in Figure 6C.

In one embodiment, system 600c allows one or more treatment parameters to be adjusted to minimize discomfort and/or pain to patients that may result from differences in skin type. Handpiece 620c may include a skin typing light source 636c for applying a multi-wavelength light to determine a patient's skin type. Although different skin typing systems may be used, in one embodiment the system 600c may determine a Fitzpatrick skin type of the patient similar to Figure 6B. Skin typing light source 636c may generate noncoherent, multi-wavelength light in visible and/or IR light ranges. A skin typing light sensor 638c may be provided to sense a portion of the light from light source 636 that is reflected from the skin of the patient.

Controller 640c may include a skin type determination unit 646c that receives data from the skin typing light sensor 638c relating to, *e.g*., the absorbance or non-absorbance of the patient's skin of particular wavelengths of light, analyzes the absorbance/non-absorbance data, and determines a skin type of the patient. Controller 640c may include logic (*e.g*., executable software or firmware code, not shown) to modify one or more aspects of the laser treatment based on the patient's skin type, or provide such data to a user, to maintaining the skin surface temperature below a desired maximum during treatment. In some embodiments, the skin type determination components (e.g., 636, 638, 346) may be omitted.

The controller 640c may also comprise a laser power-cooling ratio unit 648c. The laser power-cooling ratio unit 648c is capable of adjusting the cooling power based on the laser power and/or vice versa to provide a predetermined cooling system power to laser power ratio. For example, the controller 640c may be configured to control the treatment process such the ratio of the cooling power and the laser source power is at least 20%, or alternatively, the ratio of the cooling power and the laser source power is at least 15%. The controller 640c is preferably capable of providing numerous combinations for laser power and cooling power to provide a variety of laser power to cooling ratios. Examples of laser power and laser power-to-cooling ratios include, but are not limited to:
- laser source power of 20 Watts, cooling-to-laser power ratio of 20%
- laser source power of 20 Watts, cooling-to-laser power ratio of 15%
- laser source power of 20 Watts, cooling-to-laser power ratio of 30%
- laser source power of 20 Watts, cooling-to-laser power ratio of 35%
- laser source power of 25 Watts, cooling power 3.75 Watts
- laser source power of 30 Watts, cooling power 4.5 Watts
- laser source power of 35 Watts, cooling power 5.25 Watts
- laser source power of 40 Watts, cooling power 6 Watts
- laser source power of 50 Watts, cooling power 6.75 Watts
- laser source power of 55 Watts, cooling power 8.25 Watts
- laser source power of 60 Watts, cooling power 9 Watts
- laser source power of 70 Watts, cooling power 10.5 Watts
- laser source power of 75 Watts, cooling power 11.25 Watts

The therapeutic laser system of Figure 6C is also preferably capable of insuring that the handpiece includes a mechanism for assisting the user with properly positioning the handpiece against the skin of the patient. For example, in one embodiment, the controller 600c includes a contact sensing controller 645d. The contact sensing controller 645c is generally configured to interact with a contact sensing unit 625c to determine when the handpiece is oriented with substantially planar contact between the skin of the patient and the cooling window and/or to sense when the handpiece is pressed onto the skin of the patient with sufficient pressure and/or force to cause the TEC 630c to properly cool a first skin area in contact with the contact window 628c, and to heat the target skin areas within the first skin area to a desired temperature and with a desired pulse distribution. Contact sensing controller 645c may be configured to communicate with the user of the handpiece via a contact indicator 652c, such as by communicating that planar contact has been established and/or that sufficient pressure/force is being applied between the handpiece (e.g., via the contact window) and the skin of the patient. The contact sensing unit 625c may include a plurality of contact sensing elements 627c located on the frame 626c, which may take the form of mechanical or optical sensing elements. In some embodiments, contact sensing may be omitted from handpiece 620c.

Figures 6A, 6B and 6C illustrate a system according to certain embodiments of the invention involving cooling the skin before, during, and after pulse delivery. However, alternative embodiments of the invention include systems with no cooling of the skin, or without cooling of the skin during one or more of the periods before, during, and after delivery of the therapeutic laser pulse. Additional alternative embodiments include systems in which different cooling capacities (*i.e*., rate of heat removal from the skin) are used in the periods before, during, or after delivery of the laser pulse, and during portions of these periods.

Figure 7 is a simplified sectional view of the interior of a handpiece 700 for cooling a portion of a skin area 730 and applying laser pulses to one or more target skin areas, according to one embodiment. Laser pulses, visually shown as a laser beam 720 at an instant of time, are delivered to the handpiece 700 via an optical fiber 740 from a diode laser (*e.g*., laser 610 of Figure 6). After exiting optical fiber 740, pulses 720 pass through optical elements 770 and an aperture in a temperature detection mirror 780. Pulses 720 are redirected by a scanner 760 which may comprise a mirror, and pass through a cooling window 710 to a target skin area within a first skin area in contact with the cooling window. Scanner 760 may be controllable (*e.g*., by a motor) and repositionable laser pulses 720 are sequentially directed to a series of different target skin areas within the cooling window, without moving the cooling window 710.

Handpiece 700 also includes a thermoelectric cooler 750, which includes a heatsink portion 752 in contact with cooling window 710 to maintain the cooling window at a desired (*e.g*., programmed) temperature during contact with the first skin area. In one embodiment, cooling window 710 cools the first skin area from a first surface temperature (*e.g*., body temperature) to a second surface temperature before laser pulses 720 are applied to the skin. In one embodiment, the target skin area is cooled before, during, and after application of a laser pulse thereto.

In some embodiments, skin temperatures may be detected by infrared power or energy radiated from the skin through the cooling window 710. This infrared power or energy is reflected by scanner 760 onto temperature detector mirror 780, which focuses the infrared power on a detection element (not shown) that enables a processor to determine the temperature of a target skin area based on the infrared power from the detector mirror 780. Temperatures of a target skin area may be determined at a desired sampling rate as previously noted. Additional details on such a handpiece design are available in US Pat. No. 11,253,720, issued February 22, 2022, which is hereby incorporated in its entirety

Figure 8 is a simplified, partial exploded and external view of a handpiece 800 for cooling skin and applying laser pulses thereto. The internal components discussed in Figure 7 may be enclosed within a housing 805. A cooling window 810 (which may be the same as cooling window 710 of Figure 7) is maintained in contact with a cooling heatsink 820 (which may be the same as heatsink portion 752 of TEC 750 of Figure 7) by a window frame 830. Window frame 830 has no thermal function and merely maintains cooling window 810 in contact with heatsink portion 820 of a TEC. Additional or alternative components may be substituted for one or more of those shown in Figures 7 and 8, and other similar handpiece configurations and designs may be used to practice the inventions disclosed herein, which are limited only by the claims.

In some embodiments (not shown), a handpiece may be omitted entirely, and temperature-controlled delivery of a therapeutic pulse to a target skin area may be performed with other structures to deliver the laser pulse.

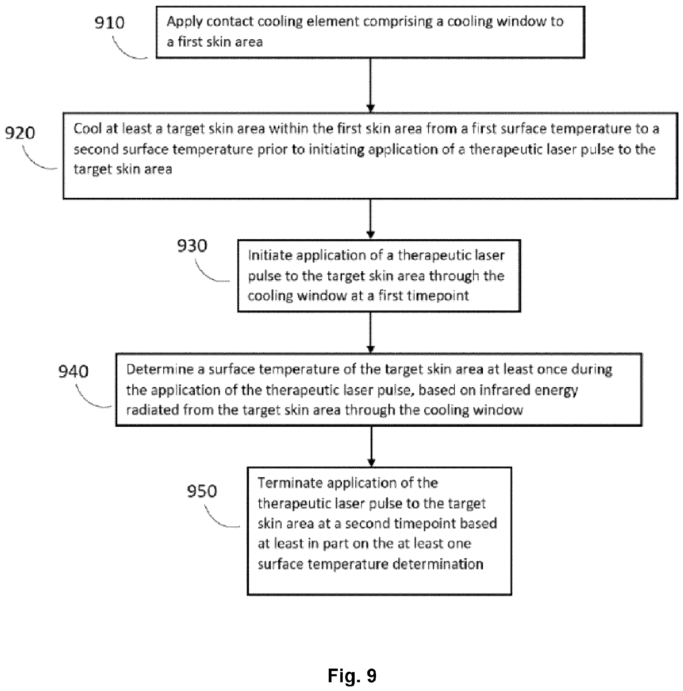

Figure 9 discloses one embodiment of a laser treatment method according to this disclosure. The method involves using a contact cooling window to cool the skin of the patient to a desired temperature, and providing a therapeutic laser pulse whose duration is determined by periodic temperature determinations or measurements during pulse delivery. The temperature determinations may comprise real-time temperature measurements used by a processor to control the duration of the laser pulse (*e.g*., based on a target skin area temperature).

The method of Figure 9 comprises applying a contact cooling element to a first skin area (910). The contact cooling element comprises a cooling window. In one embodiment, the cooling window is cooled to a reservoir temperature below a first skin temperature of the skin prior to contact with the cooling window. The cooling window is moved into contact, with the skin (preferably direct contact), and cools the skin from a first temperature to a second temperature below the first temperature. In preferred embodiments, the reservoir temperature is below the second temperature to provide a temperature difference with the skin to enable the cooling window to cool the skin surface to the second temperature relatively quickly, *e.g*., 10 sec or less, preferably 5 second or less, more preferably 3 seconds or less, more preferably about 2 seconds.

The method of Fig. 9 further comprises cooling at least a target skin area within the first skin area from a first surface temperature to a second surface temperature prior to initiating application of a therapeutic laser pulse to the target skin area (920). The application of a therapeutic laser pulse to the target skin area through the cooling window is initiated at a first timepoint (930). The method further comprises determining a surface temperature of the target skin area at least one during the application of the therapeutic laser pulse, based on infrared energy radiated from the target skin area through the cooling window (940).

In preferred, embodiments, a plurality of surface temperature determinations are made during pulse delivery. A linear or polynomial fit to the temperature data may be used to determine a rate of increase of the skin temperature caused by the delivery of the laser power to the target skin area, and the line or polynomial may be used to identify a future timepoint at which a desired temperature will be reached.

Finally, the method of Figure 9 includes terminating application of the therapeutic laser pulse to the target skin area at a second timepoint based at least in part on the at least one surface temperature determination (950). In different embodiments, the second timepoint may comprise a timepoint at which a temperature determination indicates that the surface skin temperature of the target skin area has reached or slightly exceeded a desired second temperature, or a timepoint predicted from a linear or polynomial fit to prior temperature determination data as discussed above.

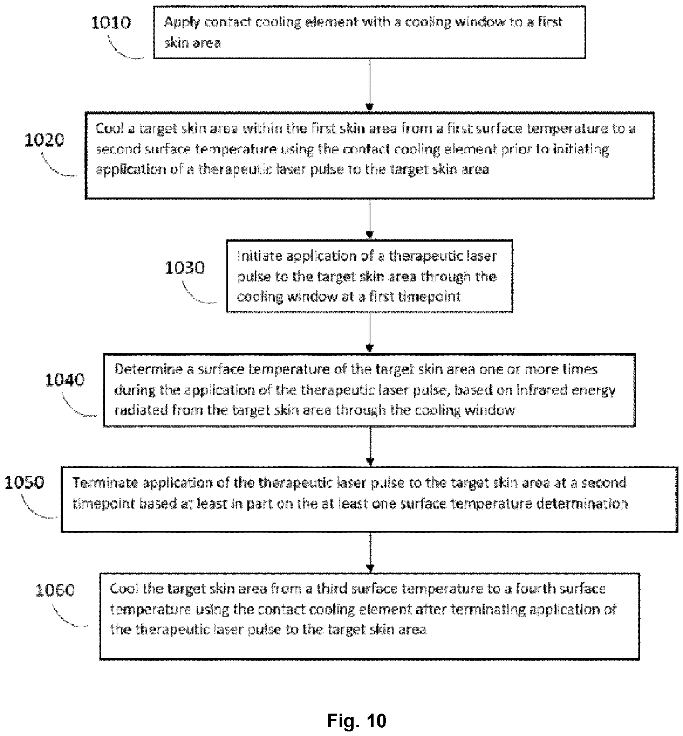

Figure 10 discloses another embodiment of a method according to the present invention. The method comprises applying a contact cooling element with a cooling window to a first skin area (1010). The method further comprises at least one cooling step, which may comprise a cooling step prior to initiating, or after terminating, the application of a therapeutic laser pulse to a target skin area within the first skin area. In the former case, the method comprises cooling a target skin area within the first skin area from a first surface temperature to a second surface temperature using the contact cooling element prior to initiating the application of a therapeutic laser pulse to the target skin area (1020). In the latter case, the method comprises cooling the target skin area from a third surface temperature (*e.g*., a temperature of the surface of the target skin area at the moment of terminating a therapeutic laser pulse) to a fourth surface temperature (*e.g*., a desired temperature below the third temperature, such as 50°C, 45°C, 40°C, or body temperature) using the contact cooling element after terminating application of the therapeutic laser pulse (1060). In one embodiment, step 1020 is omitted and step 1060 is performed. In one embodiment, step 1020 is included in the method and step 1060 is omitted. In a still further embodiment, both steps 1020 and 1060 are performed.

Referring again to Figure 10, the method further comprises initiating the application of a therapeutic laser pulse to the target skin area through the cooling window at a first timepoint (1030). After the laser pulse is initiated, the method comprises determining a surface temperature of the target skin area one or more times during the application of the laser pulse, based on infrared power radiated from the target skin area through the cooling window (1040). In preferred embodiments, a plurality of surface temperature determinations is made during pulse delivery at step 1040. In these embodiments, a linear or polynomial fit to the temperature data may be performed and used to identify/predict a future timepoint at which a desired temperature will be reached.

Finally, the method includes terminating the application of the therapeutic laser pulse at a second timepoint based on the at least one surface temperature determination (1050). In different embodiments, the second timepoint may comprise a timepoint at which a temperature determination indicates that the surface of the target skin area has reached or slightly exceeded a desired second temperature, or a timepoint predicted from a linear or polynomial fit to prior temperature determination data.

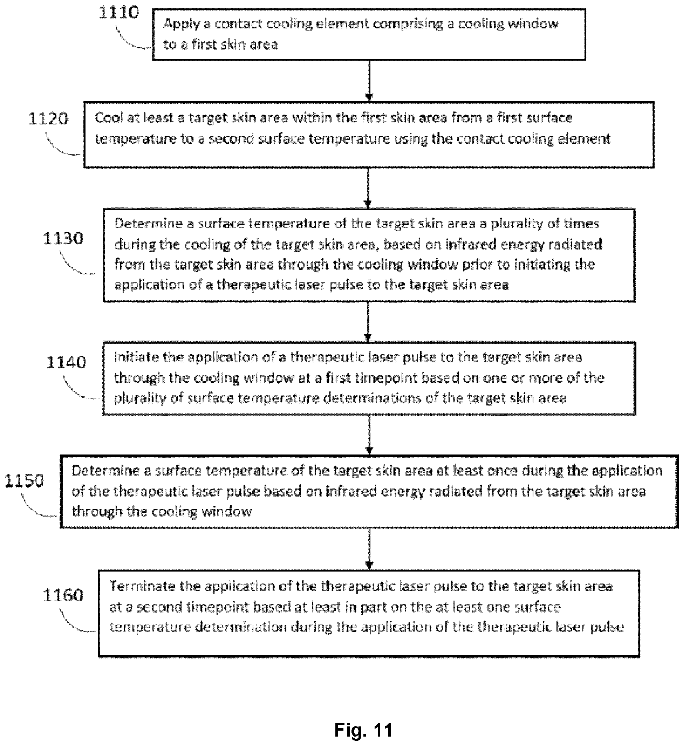

Figure 11 discloses another embodiment of a method according to the present invention. The method comprises applying a contact cooling element having a cooling window to a first skin area (1110), and cooling at least a target skin area within the first skin area from a first surface temperature to a second surface temperature using the contact cooling element (1120). In one embodiment, the ratio of the cooling power and the laser source power is at least 20%. In an alternative embodiment, the ratio of the cooling power and the laser source power is at least 15%. In one embodiment, step 1120 comprises cooling the surface temperature of the target skin area from body temperature (*e.g*., 36.5-37.5°C) to a lower temperature (*e.g*., a temperature within the range of -10°C to 20°C) that may vary depending upon factors such as the depth of the target structure , and the maximum desired temperature for tissue overlying the target structure.

The method further comprises determining, prior to initiating the application of a therapeutic laser pulse to the target skin area, a surface temperature of the target skin area a plurality of times during the cooling of the target skin area, based on infrared energy radiated from the target skin area through the cooling window (1130). As discussed in connection with Figures 6A, 7 and 8, in one embodiment the temperature may be determined by a temperature sensing control unit (*e.g*., 644, Figure 6A), which processes temperature sensing data from *e.g*., a temperature sensing mirror 780, Figure 7 focusing infrared energy on a detection element such as a photodiode having a desired sampling interval, *e.g*., 0.5-2.0 msec. In one embodiment, the photodiode is sensitive to light within a wavelength range of from about 2-40 microns and is insensitive to light at the laser wavelength range. In other embodiments, a CCD (charge-coupled device) or CMOS (complementary metal oxide semiconductor) light sensor may be used as the detection element. In a particular embodiment, the detection element may comprise at model IR1011 sensor, available from AKM Semiconductor, Inc., San Jose, CA, although it will be appreciated that other sensors may be used in different embodiments. The detection element may be selected based in part on the material used for the cooling window 1130. Regardless of the type of temperature sensing structure(s) used to sense the surface temperature of the target skin area, the temperature determinations are performed a plurality of times from infrared energy radiated through the cooling window.

Referring again to Figure 11, the method further comprises, at step 1140, initiating the application of a therapeutic laser pulse to the target skin area through the cooling window at a first timepoint based on one or more of the plurality of surface temperature determinations from step 1130. In one embodiment, the first timepoint may one at which a surface temperature determination indicates that the target skin area has been cooled to the second surface temperature. In one embodiment, the first timepoint may be a predicted timepoint, based on one or more of the temperature determinations, of when the target skin area will reach the second surface temperature.

After the therapeutic laser pulse is initiated, the method comprises determining a surface temperature of the target skin area at least once during the application of the therapeutic laser pulse, based on infrared energy radiated from the target skin area through the cooling window (1150). In preferred embodiments, a plurality of surface temperature determinations is made during pulse delivery.

Finally, the method includes terminating the application of the therapeutic laser pulse to the target skin area at a second timepoint based on the at least one surface temperature determination during the application of the therapeutic laser pulse (1160). In different embodiments, the second timepoint may comprise a timepoint at which a temperature determination indicates that the surface skin temperature of the target skin area has reached or slightly exceeded a desired temperature, or a timepoint predicted from a linear or polynomial fit to prior temperature determinations.

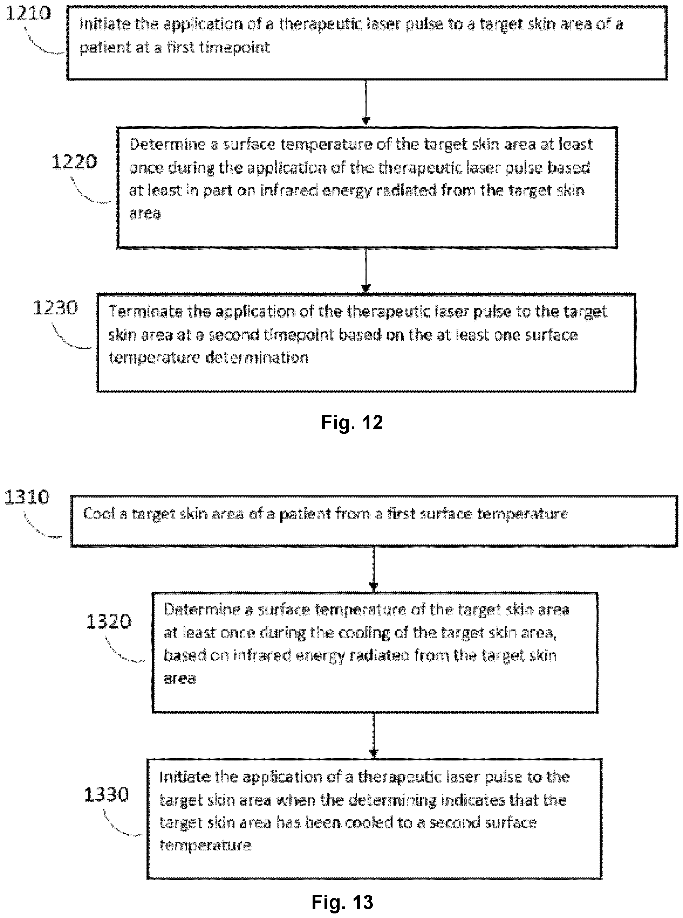

In one aspect, the invention comprises methods of treating a patient by controlling the duration of a laser pulse without a required cooling step. Figure 12 discloses one embodiment of such a method. The method comprises initiating the application of a therapeutic laser pulse to a target skin area of a patient at a first timepoint (1210). After the pulse is initiated, the method further includes determining a surface temperature of the target skin area at least once during the application of the therapeutic laser pulse based on infrared energy radiated from the target skin area (1220). The determination(s) of surface temperature may be performed as described in the discussion of Figure 11. Finally, the method includes terminating the application of the therapeutic laser pulse at a second timepoint based on the at least one surface temperature determination (1230).

In one aspect, the invention comprises methods of treating a patient by initiating a therapeutic laser pulse when the skin has been pre-cooled to a desired temperature. Figure 13 discloses one embodiment of such a method. The method comprises cooling a target skin area of a patient from a first surface temperature (1310), and determining a surface temperature of the target skin area at least once during the cooling of the target skin area based on infrared power radiated from the target skin area (1320). In one embodiment, the ratio of the cooling power and the laser source power is at least 20%. In an alternative embodiment, the ratio of the cooling power and the laser source power is at least 15% Finally, the method comprises initiating the application of a therapeutic laser pulse to the target skin area when the determining step (*i.e*., step 1320) indicates that the target skin area has been cooled to a second surface temperature.

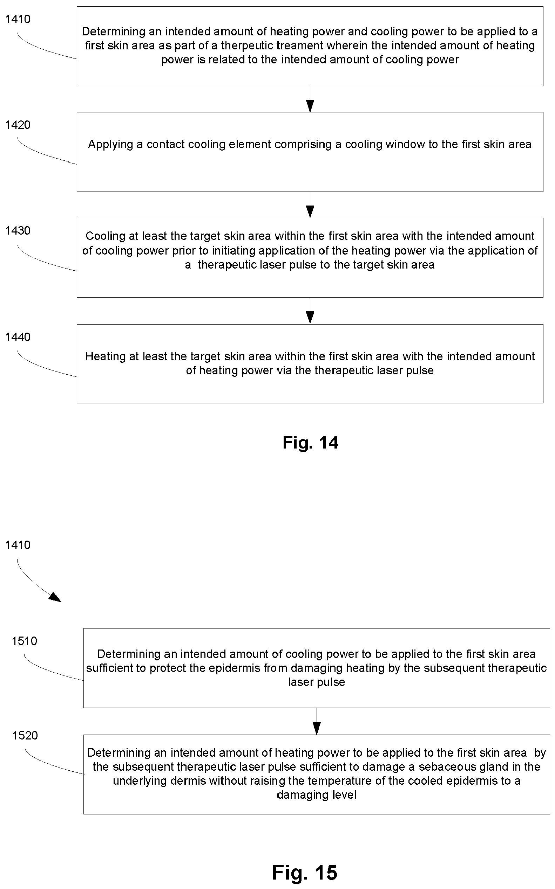

In another aspect, the invention comprises methods of treating a patient by initiating a therapeutic laser pulse when the skin has been pre-cooled with a desired amount of cooling power. Figure 14 discloses one embodiment of such a method. The method comprises determining an intended heating power and cooling power to be applied to a first skin area as part of a therapeutic laser treatment wherein the intended heating power is related to the intended cooling power (1410). Thereafter, as discussed more fully previously, a contact cooling element comprising a cooling window is applied to a first skin area (1420) and cools at least a target skin area within the first skin area with the intended cooling power prior to initiating application of a therapeutic laser pulse to the target skin area using a laser source having the intended heating power (1430). Subsequently, the therapeutic laser pulse heats at least the target skin area within the first skin area with the laser source having the intended amount of heating power (1440).

Figure 15 discloses a more detailed embodiment of the method set forth in step (1410) of Figure 14. The method comprises two interrelated steps. First, the method involves determining an intended amount of cooling power to be applied to the first skin area sufficient to protect the epidermis from damaging heating by a subsequent therapeutic laser pulse applied to a target skin area within the first skin area (1510). Second, the method involves determining an intended amount of heating power to be applied to the first skin area by the subsequent therapeutic laser pulse sufficient to damage a sebaceous gland in the target skin area dermis without raising the temperature of the cooled epidermis to a damaging level (1520). That is, as additional precooling is applied, additional heating should be administered to ensure that the temperature of the underlying sebaceous gland reaches a temperature sufficient to damage the sebaceous gland, and vice versa.

In one embodiment, the preselected amount of cooling power used to pre-cool the target skin area is about 20% of the preselected amount of heating power used to subsequently heat the target skin area. In one embodiment, the laser pulse used to heat the target skin area is a tophat pulse (*i.e*., having a uniform intensity profile over the covered area) and has one or more parameters defining the pulse selected from a wavelength of 1727.5 nm, a pulse duration of about 30 msec, a beam diameter of about 2.8 mm, a power of about 75 W, a pulse energy of about 2.25 J, and a fluence of about 37 J/cm². In one embodiment, the cooling power applied to the target skin area is produced by a thermoelectric cooler (TEC). Those skilled in the art will readily appreciate that other known cooling power sources could be substituted. Exemplary information regarding the structure and operation of TECs may be found, for example, on the Internet at https://www.tec-microsystems.com/faq/thermoelectic-coolers-intro.html.

TECs are known to have a thermoelectric cooling capacity (Q). In one embodiment, the thermoelectric cooling capacity (Q) is selected to be about 20% of the heating power to be supplied by the laser source generating the therapeutic laser pulse(s). In a particular example the power of the laser is selected to be 75 W, and thus the cooling capacity of the TE Cooler should be selected to be at least about 15 W (75 x 0.2). Similarly, if the power of the laser is selected to be about 100 W, then the cooling capacity of the TE Cooler should be selected to be at least about 20 W (75 x 0.2).

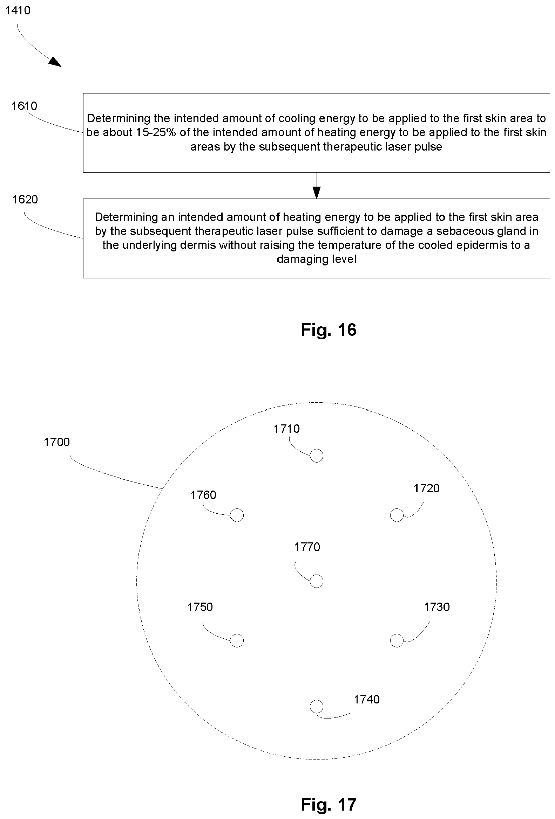

Those skilled in the art will appreciate that variations in the color, thickness and composition of the epidermis may permit variations in the relationship between the cooling power used to precool the first skin area and the amount of heating power used to subsequently heat a target skin area within the first skin area. That is, depending on the characteristics of the patient's skin, the ratio of cooling power to heating power may vary between 15% and 25%, depending upon, for example, a Fitzpatrick score assigned to the patient's skin type via the skin type determination unit 646. In other examples, the ratio of cooling to heating power may vary between 15% and 35%, Figure 16 illustrates an alternative embodiment of step 1410 reflecting variations in the ratio of cooling to heating to accommodate differences in the patient's skin. In particular, step 1610 demonstrates that the cooling to heating power ratio may be varied in the range of 15-25% owing to the fact that more heat will be absorbed in the overlying epidermis in patients with high melanin, potentially causing overheating and damage to the epidermis unless that ratio is increased. On the other hand, patients with lower levels of melanin may experience less heating of the epidermis, and thus, may tolerate a decreased cooling to heating power ratio owing to a reduced temperature rise in the epidermis.

Those skilled in the art will appreciate that the ratio may be increased by changing a variety of parameters. For example, the cooling power applied to the first skin area may be increased, the heating power may be reduced, or a combination of both. Similarly, the ratio may be decreased by decreasing the cooling power applied to the first skin area, increasing the heating power, or a combination of both.

In some embodiments, it may be useful to apply the laser as a series of pulses dispersed over a preselected area (i.e., a first skin area including one or more target skin areas). For example, FIG 17 stylistically illustrates a first area 1700 that is generally circular in shape. In this embodiment the laser is aimed at a series of six targets 1710-1760 that roughly approximates the vertices of a hexagon and a seventh target 1770 at about the geometric center of the hexagon. This pattern distributes the laser pulses substantially evenly to achieve a substantially even distribution of thermal damage resulting from the laser pulses within the circular first skin area 1700. In one embodiment, the spacing between the target skin areas is substantially similar at a first preferred range of about 250 microns (µm) to about 500 µm, but can be varied to be between 0.0 µm to about 1.0 mm. In some embodiments, the spacing between the target areas may be varied depending upon the physical characteristics of the patient's skin, such as by determining a Fitzpatrick score. In one embodiment, the series of seven laser pulses are each about 30 msec in duration with pauses of about 15 msec therebetween.

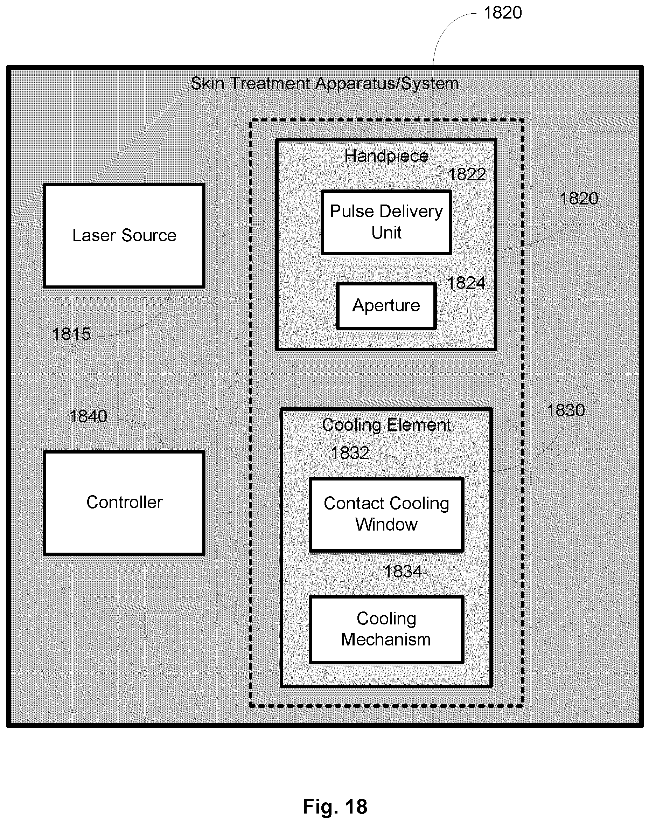

Fig. 18 illustrates a stylized block diagram depiction of a skin treatment system according to some embodiments herein. The skin treatment apparatus/system 1820 is capable of providing therapeutic laser pulses for applications to one or more target skin areas, such as those shown in Fig. 17. The skin treatment apparatus/system 1820 comprises a laser source 1815, which may be similar to the laser source(s) described above (e.g., in connection with Fig. 17, or diode laser 610, Figure 6A). For example, in one embodiment the laser source 1815 is capable of providing a pulsed laser light at a wavelength of between about 2287-2318 nm.

**The** skin treatment apparatus/system 1820 comprises a handpiece 1820 operatively coupled to laser source 1815. "Operatively coupled" may include optical coupling and/or other types of coupling, including electrical coupling. The handpiece 1820 is configured to deliver laser pulses to target skin area(s) of the patient, and may comprise a pulse delivery unit 1822, which is adapted to direct laser pulses from laser source 1815 to the target skin area(s) via an aperture 1824.

Skin treatment apparatus/system 1820 also comprises a cooling system (or element) 1830. In one embodiment, the cooling system 1830 may be a part of the handpiece 1820, while in other embodiments, the cooling system 1830 may be separate from the handpiece.

**The** cooling element is capable of providing cooling of the target skin area. The cooling system 1830 comprises a contact cooling window 1832, which may be operatively coupled with a cooling mechanism 1834 that may act as a cooling source. The cooling mechanism 1834 may comprise a thermoelectric cooler (TEC), as described above. The cooling process may be controlled such that the ratio of the cooling power of system 1830 and the laser source power is at least 20%. Contact cooling window 1832 may comprise any of a variety of laser- and IR-transmissive materials, including sapphire, ZnS, diamond, ZnSe, and other materials. In alternative embodiments (not shown), the cooling element may comprise components or structures in addition to cooling window 628, such as a copper (or other material having a high thermal conductivity) cooling element that is not light-transmissive to provide additional cooling capacity.

The skin treatment apparatus/system 1820 may also comprise a controller 1840 configured to control various operations of the skin treatment apparatus/system 1820. In some embodiments, controller 1840 may be similar to the controller described above with reference to Figs. 6A-6C. Controller 1840 is capable of delivering laser power and cooling power at a predetermined ratio. For example, the controller 1840 may control the ratio of the cooling power and the laser source power to be at least 20% in one embodiment, while in an alternative embodiment, this ratio is at least 15%. Other combinations of cooling power and laser power may be applied to achieve various ratios as described above.

In various embodiments, the present invention relates to the subject matter of the following numbered paragraphs.
101. A method of controlling a duration of a therapeutic laser pulse to the skin of a patient, the method comprising:
   initiating, at a first timepoint, the application of a therapeutic laser pulse to a target skin area of the patient;
   applying a contact cooling element comprising a cooling window to the target skin area proximate to the target skin area, wherein the ratio of the cooling power and the laser source power is at least one of at least 20% or at least 15%;
   determining a surface temperature of the target skin area at least once during the application of the therapeutic laser pulse based at least in part on infrared power radiated from the target skin area wherein the ratio of the cooling power and the laser source power is one of at least 20% or at least 15%; and
   terminating the application of the therapeutic laser pulse to the target skin area at a second timepoint based on the at least one surface temperature determination.
102. The method of 101, further comprising:
   applying a contact cooling element comprising a cooling window to a first skin area proximate to the target skin area; and
   cooling at least the target skin area from a first surface temperature to a second surface temperature, wherein the ratio of the cooling power and the laser source power is at least one of at least 20% or at least 15%.
103. The method of 102, wherein the cooling window comprises a material selected from sapphire, ZnS, diamond, ZnSe, and a different thermally conductive material that is transmissive to infrared light.
104. The method of 102, wherein the step of cooling at least the target skin area to the second surface temperature is performed prior to initiating the application of the therapeutic laser pulse to the target skin area.
105. The method of 102, wherein cooling at least the target skin area comprises cooling the cooling window using a cooling medium selected from the group consisting of water, a halogenated hydrocarbon refrigerant, and air.
106. The method of 102, wherein cooling at least the target skin area comprises cooling the cooling window using a thermoelectric cooler.
107. The method of 101, further comprising cooling at least the target skin area from a first surface temperature to a second surface temperature.
108. The method of 107, wherein cooling at least the target skin area comprises cooling at least the target skin area, prior to initiating the application of the therapeutic laser pulse, from a first surface temperature of body temperature to a second surface temperature within the range of -10°C to 20°C.
109. The method of 107, wherein cooling at least the target skin area comprises cooling at least the target skin area, prior to initiating the application of the therapeutic laser pulse, from a first surface temperature to a second surface temperature within the range of -5°C to 10°C.
110. The method of 107, wherein cooling at least a target skin area comprises cooling the surface of the target skin area, prior to initiating the application of the therapeutic laser pulse, from a first surface temperature to a second surface temperature within the range of -5°C to 5°C.
111. The method of 107, wherein cooling at least the target skin area comprises one of
   cooling the target skin area from the first surface temperature to the second surface temperature prior to initiating the application of the therapeutic laser pulse; and
   cooling the target skin area from the first surface temperature to the second surface temperature after terminating the application of the therapeutic laser pulse.
112. The method of 107, wherein cooling at least the first target skin area from a first surface temperature to a second surface temperature comprises cooling the target skin area to the second surface temperature prior to initiating the application of the therapeutic laser pulse, the method further comprising
   cooling the target skin area from a third surface temperature to a fourth surface temperature after terminating the application of the therapeutic laser pulse.
113. The method of 112, wherein the third surface temperature is user-selectable.
114. The method of 112, wherein at least one of the third surface temperature and the fourth surface temperature is user-selectable.
115. The method of 101, further comprising:
   determining the pulse duration of the therapeutic laser pulse as the difference between the first and second timepoint;
   applying a subsequent therapeutic laser pulse to the of controlling a duration of a therapeutic laser pulse to the skin of a patient, the method comprising:
      initiating, at a first timepoint, the application of a therapeutic laser pulse to a target skin area of the patient;
      determining a surface temperature of the target skin area at least once during the application of the therapeutic laser pulse based at least in part on infrared power or energy radiated from the target skin area; and
      terminating the application of the therapeutic laser pulse to the target skin area at a second timepoint based on the at least one surface temperature determination.
116. The method of 101, wherein determining a surface temperature of the target skin area at least once during the application of the therapeutic laser pulse comprises determining a surface temperature of the target skin area a plurality of times during the application of the therapeutic laser pulse at a predetermined temperature sampling time interval of 100 msec or less.
117. The method of 116, wherein the predetermined temperature sampling time interval is a time interval within the range of 0.01-10.0 msec.
118. The method of 101, wherein initiating the application of a therapeutic lase pulse comprises initiating a laser pulse at a power density of at least 100 W/cm2.
119. The method of 101, wherein initiating the application of a therapeutic laser pulse comprises initiating a laser pulse having a power fluence of at least 2 J/cm2.
120. The method of 119, wherein the therapeutic laser pulse has a power fluence within the range of 2-100 J/cm2.
121. The method of 120, wherein the therapeutic laser pulse has a power fluence within the range of 2-40 J/cm2.
201. A method of treating the skin of a patient with a therapeutic laser pulse from a therapeutic laser source having a laser source power, the method comprising:
   a) applying a contact cooling element comprising a cooling window to a first skin area of the patient;
   b) cooling at least a target skin area within the first skin area from a first surface temperature using the contact cooling element, wherein the ratio of the cooling power and the laser source power is at least one of at least 20% or at least 15%;
   c) determining a surface temperature of the target skin area a plurality of times during the application of the contact cooling element to the first skin area, wherein each of said surface temperature determinations is based on infrared power radiated from the target skin area through the cooling window prior to initiating the application of a therapeutic laser pulse to the target skin area;
   d) initiating the application of a therapeutic laser pulse to the target skin area through the cooling window at a first timepoint based at least in part on one or more of the plurality of surface temperature determinations of the target skin area.
202. The method of 201, further comprising:
   e) determining a surface temperature of the target skin area at least once during the application of the therapeutic laser pulse, based on infrared power radiated from the target skin area through the cooling window; and
   f) terminating the application of the therapeutic laser pulse to the target skin area at a second timepoint based on the at least one surface temperature determination.
301. A method of treating the skin of a patient with a therapeutic laser pulse, the method comprising:
   a) cooling a target skin area of the patient from a first surface temperature, wherein the ratio of the cooling power and the laser source power is at least one of at least 20% or at least 15%;
   b) determining a surface temperature of the target skin area a plurality of times during the cooling of the target skin area, wherein the determining is based on infrared power radiated from the target skin area;
   c) initiating the application of a therapeutic laser pulse to the target skin area when the determining indicates that the target skin area has been cooled to a second surface temperature.
302. The method of 301, further comprising:
   d) determining a surface temperature of the target skin area a plurality of times during the application of the therapeutic laser pulse to the target skin area, wherein the determining is based on infrared power radiated from the target skin area.
303. The method of 301, further comprising:
   d) characterizing the patient's skin; and
   e) determining a target second surface temperature based on the characterizing.
304. The method of 303, wherein characterizing the patient's skin comprises determining a melanin content of at least a portion of the patient's skin, and wherein determining a target second surface temperature comprises selecting a lower target second surface temperature the higher the patient's melanin content.
305. The method of 303, wherein characterizing the patient's skin comprises determining a Fitzpatrick score indicative of the skin type of the patient, and wherein determining a second target surface temperature comprises selecting a lower target second surface temperature for higher Fitzpatrick scores.

The application provides the following further embodiments.
1. A method of treating the skin of a patient with a therapeutic laser pulse from a laser source having a laser source power, the method comprising:
   a) using a contact cooling system comprising a contact cooling window and a thermoelectric cooler (TEC), applying the contact cooling window to a first skin area of the patient, wherein the contact cooling window is coupled to a handpiece adapted to be held by a user;
   b) cooling at least one target skin area within the first skin area using the contact cooling system, wherein the cooling power is provided by the TEC, which is thermally coupled to the contact cooling window and has a cooling power of at least 4 Watts;
   c) applying one or more therapeutic laser pulses to the at least one target skin area through the cooling window, wherein the one or more therapeutic laser pulses are generated using a laser source having a laser source power of at least 20 Watts, wherein the ratio of the cooling power and the laser source power is at least 20%.
2. The method of embodiment 1, wherein applying one or more therapeutic laser pulses comprises applying one or more therapeutic laser pulses having a wavelength of 1200-2400 nm.
3. The method of embodiment 1, wherein applying one or more therapeutic laser pulses comprises applying one or more therapeutic laser pulses having a wavelength of 1200-1800 nm.
4. The method of embodiment 1, wherein the cooling window comprises a material selected from sapphire, ZnS, diamond, ZnSe, and a thermally conductive material.
5. The method of embodiment 1, wherein the ratio of the cooling power and the laser power may be adjusted based on at least a skin type of the patient.
6. The method of embodiment 1, wherein the laser source power is at least 20 Watts, and the ratio of the cooling power and the laser source power is at least 30%.
7. The method of embodiment 1, wherein the cooling power is at least 5 Watts, and the laser source power is at least 25 Watts.
8. The method of embodiment 1, wherein the cooling power is at least 10 Watts, and the laser source power is at least 50 Watts.
9. The method of embodiment 8, wherein the laser source power is at least 50 Watts, and the ratio of the cooling power and the laser source power is at least 30%.
10. The method of embodiment 1, wherein the laser source power is at least 20 Watts, and the ratio of the cooling power and the laser source power is at least 35%.
11. The method of embodiment 1, wherein the laser source power is about 100 Watts.
12. The method of embodiment 1, further comprising repeating steps b-c one or more times, wherein the target skin area for each repetition of steps b-c comprises a target skin area within the first skin area that is non-contiguous with every other target skin area for each sequence of steps b-c.
13. The method of embodiment 12, wherein repeating steps b-c one or more times comprises using a mirror to direct the laser pulse for each repetition of steps b-c to a different target area within the first skin area that is non-contiguous with every other target area for each sequence of steps b-c.
14. The method of embodiment 1, wherein initiating the application of a therapeutic laser pulse comprises initiating a laser pulse from a diode laser, and wherein the pulse has an energy fluence of at least 2 J/cm².
15. The method of embodiment 1, wherein initiating the application of a therapeutic laser pulse comprises initiating the application of optical energy having a wavelength of within a range of from about 1700 nm to about 1740 nm.
16. The method of embodiment 15, wherein the wavelength is a wavelength in the range of 1726-1727.5 nm.
17. A method of treating the skin of a patient with a therapeutic laser pulse using a laser source having a laser source power, the method comprising:
   a) applying a contact cooling unit comprising a cooling window to a first skin area of the patient;
   b) cooling at least a target skin area within the first skin area prior to initiating an application of a therapeutic laser pulse to the target skin area, wherein the cooling power applied to the target skin area is a first preselected setpoint; and
   c) applying a therapeutic laser pulse to the target skin area through the cooling window at a first timepoint, wherein the laser source power is a second preselected setpoint, the first preselected setpoint having a ratio of at least 20% of the second preselected setpoint.
18. The method of embodiment 17, wherein cooling at least the target skin area comprises cooling the first skin area in contact with the cooling window using a thermoelectric cooler thermally coupled to the cooling window.
19. The method of embodiment 17, further comprising:
   d) determining a skin type of the patient, wherein the skin type is indicative of a melanin concentration of the patient's skin.
20. The method of embodiment 17, wherein cooling at least a target skin area within the first skin area comprises cooling the target skin area via the application of a preselected magnitude of cooling power based on the determined skin type of the patient.
21. The method of embodiment 17, wherein determining a skin type of the patient comprises:
   a) applying light from a multi-wavelength light source to a second skin area;
   b) analyzing reflected multi-wavelength light from the second skin area; and
   c) determining a skin type of the patient based on the analyzed reflected multi-wavelength light.
22. The method of embodiment 17, wherein the patient is a patient having one of acne vulgaris or hyperhidrosis.

The particular embodiments disclosed and discussed above are illustrative only, as the invention may be modified and practiced in different but equivalent manners apparent to those skilled in the art having the benefit of the teachings herein. Embodiments of the present invention disclosed and claimed herein may be made and executed without undue experimentation with the benefit of the present disclosure. While the invention has been described in terms of particular embodiments, it will be apparent to those of skill in the art that variations may be applied to systems and apparatus described herein without departing from the concept and scope of the invention. Examples are all intended to be non-limiting. It is therefore evident that the particular embodiments disclosed above may be altered or modified and all such variations are considered within the scope of the invention, which are limited only by the scope of the claims.

## Claims

1. A therapeutic laser system for treating the skin of a patient with a therapeutic laser pulse from a laser source having a laser source power, the therapeutic laser system being configured to:
a) use a contact cooling system comprising a contact cooling window and a thermoelectric cooler (TEC), apply the contact cooling window to a first skin area of the patient, wherein the contact cooling window is coupled to a handpiece adapted to be held by a user;
b) cool at least one target skin area within the first skin area using the contact cooling system, wherein the cooling power is provided by the TEC, which is thermally coupled to the contact cooling window and has a cooling power of at least 4 Watts;
c) apply one or more therapeutic laser pulses to the at least one target skin area through the cooling window, wherein the therapeutic laser system is configured to generate the one or more therapeutic laser pulses using a laser source having a laser source power of at least 20 Watts, wherein the ratio of the cooling power and the laser source power is at least 20%.

2. The therapeutic laser system of claim 1, wherein the therapeutic laser system is configured to apply one or more therapeutic laser pulses having a wavelength of 1200-2400 nm, in particular a wavelength of 1200-1800 nm.

3. The therapeutic laser system of claim 1 or 2, wherein the cooling window comprises a material selected from sapphire, ZnS, diamond, ZnSe, and a thermally conductive material.

4. The therapeutic laser system of any one of claims 1 to 3, wherein the ratio of the cooling power and the laser power may be adjusted based on at least a skin type of the patient.

5. The therapeutic laser system of any one of claims 1 to 4, wherein the laser source power is at least 20 Watts, and the ratio of the cooling power and the laser source power is at least 30%, in particular at least 35%.

6. The therapeutic laser system of any one of claims 1 to 5, wherein the cooling power is at least 5 Watts, and the laser source power is at least 25 Watts, in particular, wherein the cooling power is at least 10 Watts, and the laser source power is at least 50 Watts.

7. The therapeutic laser system of any one of claims 1 to 6, wherein the laser source power is about 100 Watts.

8. The therapeutic laser system of any one of claims 1 to 7, wherein the therapeutic laser system is configured to repeat steps b-c one or more times, wherein the target skin area for each repetition of steps b-c comprises a target skin area within the first skin area that is non-contiguous with every other target skin area for each sequence of steps b-c.

9. The therapeutic laser system of claim 8, wherein the therapeutic laser system is configured to repeat steps b-c one or more times by using a mirror to direct the laser pulse for each repetition of steps b-c to a different target area within the first skin area that is non-contiguous with every other target area for each sequence of steps b-c.

10. The therapeutic laser system of any one of claims 1 to 9, wherein the therapeutic laser system is configured to initiate application of a therapeutic laser pulse by initiating a laser pulse from a diode laser, and wherein the pulse has an energy fluence of at least 2 J/cm².

11. The therapeutic laser system of any one of claims 1 to 10, wherein the therapeutic laser system is configured to initiate application of a therapeutic laser pulse by initiating the application of optical energy having a wavelength of within a range of from about 1700 nm to about 1740 nm, in particular, wherein the wavelength is a wavelength in the range of 1726-1727.5 nm.

12. A therapeutic laser system configured to treat the skin of a patient with a therapeutic laser pulse using a laser source having a laser source power, the therapeutic laser system being configured to:
a) apply a contact cooling unit comprising a cooling window to a first skin area of the patient;
b) cool at least a target skin area within the first skin area prior to initiat an application of a therapeutic laser pulse to the target skin area, wherein the cooling power applied to the target skin area is a first preselected setpoint; and
c) apply a therapeutic laser pulse to the target skin area through the cooling window at a first timepoint, wherein the laser source power is a second preselected setpoint, the first preselected setpoint having a ratio of at least 20% of the second preselected setpoint.

13. The therapeutic laser system of claim 12, wherein the therapeutic laser system is configured to cool at least the target skin area by cooling the first skin area in contact with the cooling window using a thermoelectric cooler thermally coupled to the cooling window and/or wherein the therapeutic laser system is configured to cool at least a target skin area within the first skin area by cooling the target skin area via the application of a preselected magnitude of cooling power based on the determined skin type of the patient.

14. The therapeutic laser system of claim 12 or 13, wherein the therapeutic laser system is configured to:
d) determine a skin type of the patient, wherein the skin type is indicative of a melanin concentration of the patient's skin.

15. The therapeutic laser system of any one of claims 12 to 14, wherein the therapeutic laser system is configured to determine a skin type of the patient by:
a) applying light from a multi-wavelength light source to a second skin area;
b) analyzing reflected multi-wavelength light from the second skin area; and
c) determining a skin type of the patient based on the analyzed reflected multi-wavelength light.
